# EUROPEAN PATENT SPECIFICATION

(11) **EP 4 132 503 B1**
(45) Date of publication and mention of the grant of the patent: **22.04.2026**
(21) Application number: 21785329.0
(22) Date of filing: 09.04.2021
(51) Int. Cl.: A61K 31/195, A61P 31/14

(54) **PROBENECID FOR USE IN THE TREATMENT OF SARS-CO-V2 INFECTIONS**
PROBENECID ZUR VERWENDUNG IN DER BEHANDLUNG VON SARS-CO-V2-INFEKTIONEN
PROBENECID POUR SON UTILISATION DANS UN MÉTHODE DE TRAITEMENT D'UNE INFECTION À SARS-CO-V2

(30) Priority: 10.04.2020 US 202063008624 P; 11.05.2020 US 202063023021 P; 15.05.2020 US 202016875487; 15.10.2020 EP 20202059; 19.02.2021 US 202163151551 P
(43) Date of publication of application: 15.02.2023
(73) Proprietor: University of Georgia Research Foundation, Inc., Athens, Georgia 30602 (US)
(72) Inventor: TRIPP, Ralph A., Athens, Georgia 30602 (US); MURRAY, Jackelyn, Athens, Georgia 30602 (US); HOGAN, Robert Jeff, Athens, Georgia 30602 (US)
(74) Representative: Potter Clarkson
(86) International application number: PCT/US2021/026588
(87) International publication number: WO 2021/207606

(56) References cited:
- WO-A1-2020/052677
- WO-A1-2020/247665
- US-A1- 2005 112 554
- US-A1- 2013 136 770
- US-A1- 2013 280 806
- US-A1- 2019 374 516
- XORTX THERAPEUTICS INC: "XORTX Launches XRx-101, A New Program to Treat Coronavirus COVID-19 Infection", 16 March 2020 (2020-03-16), XP002802384, Retrieved from the Internet <URL:https://www.biospace.com/article/releases/xortx-launches-xrx-101-a-new-program-to-treat-coronavirus-covid-19-infection/> [retrieved on 20210316]
- CHENG YICHUN ET AL: "Kidney impairment is associated with in-hospital death of COVID19 patients", MEDRXIV, 20 February 2020 (2020-02-20), XP055785781, Retrieved from the Internet <URL:https://www.medrxiv.org/content/10.1101/2020.02.18.20023242v1.full.pdf> [retrieved on 20210315], DOI: 10.1101/2020.02.18.20023242
- OLIVIA PERWITASARI ET AL: "ABSTRACT", ANTIMICROBIAL AGENTS AND CHEMOTHERAPY, vol. 57, no. 1, 5 November 2012 (2012-11-05), US, pages 475 - 483, XP055449022, ISSN: 0066-4804, DOI: 10.1128/AAC.01532-12
- SAMSAMI MAJID, ZEBARJADI BAGHERPOUR JAVAD, NEMATIHONAR BEHZAD, TAHMASBI HAMED: "COVID-19 Pneumonia in Asymptomatic Trauma Patients; Report of 8 Cases", ARCHIVES OF ACADEMIC EMERGENCY MEDICINE, SHAHID BEHESHTI UNIVERSITY OF MEDICAL SCIENCES, IRAN, vol. 8, no. 1, 1 January 2020 (2020-01-01), Iran, pages e46, XP055866199
- WEILONG SHANG, YANG YI, RAO YIFAN, RAO XIANCAI: "The outbreak of SARS-CoV-2 pneumonia calls for viral vaccines", NPJ VACCINES, vol. 5, no. 1, 6 March 2020 (2020-03-06), XP055727316, DOI: 10.1038/s41541-020-0170-0
- ANONYMOUS: "Alberta COVID Variants Surveillance Plan (version 4)", ALBERTA PRECISION LABORATORIES, 26 January 2020 (2020-01-26), pages 1 - 8, XP055866205, Retrieved from the Internet <URL:https://www.departmentofmedicine.com/meoc/covid-alberta-variants-surveillance-plan.pdf> [retrieved on 20211126]
- AHMED SYED FARAZ, QUADEER AHMED A., MCKAY MATTHEW R.: "Preliminary Identification of Potential Vaccine Targets for the COVID-19 Coronavirus (SARS-CoV-2) Based on SARS-CoV Immunological Studies", VIRUSES, MDPI, CH, vol. 12, no. 3, 25 February 2020 (2020-02-25), CH , pages 1 - 15, XP055823903, ISSN: 1999-4915, DOI: 10.3390/v12030254
- SHANG JIAN; YE GANG; SHI KE; WAN YUSHUN; LUO CHUMING; AIHARA HIDEKI; GENG QIBIN; AUERBACH ASHLEY; LI FANG: "Structural basis of receptor recognition by SARS-CoV-2", NATURE, NATURE PUBLISHING GROUP UK, LONDON, vol. 581, no. 7807, 30 March 2020 (2020-03-30), London, pages 221 - 224, XP037182125, ISSN: 0028-0836, DOI: 10.1038/s41586-020-2179-y
- MURRAY JACKELYN, HOGAN ROBERT J., MARTIN DAVID E., BLAHUNKA KATHY, SANCILLO FRED, BALYAN RAJIV, LOVERN MARK, STILL RICHARD, TRIPP : "Probenecid Inhibits SARS-CoV-2 Replication In Vivo and In Vitro", BIORXIV, 21 May 2021 (2021-05-21), XP055860520, Retrieved from the Internet <URL:https://www.biorxiv.org/content/10.1101/2021.05.21.445119v1.full.pdf> [retrieved on 20211111], DOI: 10.1101/2021.05.21.445119

## Description

### FIELD OF THE INVENTION

The field of the invention generally related to compositions and methods for the treatment and prevention of a severe acute respiratory syndrome coronavirus (SARS-CoV-2) infection.

### BACKGROUND OF THE INVENTION

A seventh human coronavirus was recently identified in Wuhan, China (Coronaviridae Study Group of the International Committee on Taxonomy of Viruses, Nat Microbiol 2020. DOI: 10.1038/s41564-020-0695-z, WHO "Pneumonia of unknown cause - China;" World Health Organization: Online, 2020). Initially described as COVID-19 after its discovery in December 2019, this virus has now been classified as a betacoronavirus within the same species as the severe acute respiratory syndrome coronavirus (SARS-CoV), which was responsible for a pandemic in 2002-2003 (Coronaviridae Study Group of the International Committee on Taxonomy of Viruses, Nat Microbiol 2020. DOI: 10.1038/s41564-020-0695-z; Ratia, et al., Proc Natl Acad Sci U S A 2008, 105 (42), 16119-24. DOI: 10.1073/pnas.0805240105; Karim, et al., MedRxiv, 5(4):536-44 (2020), DOI: 10.1101/2020.05.26.20104497). Hence, COVID-19 has now been classified as severe acute respiratory syndrome coronavirus 2 (SARS-CoV-2). Although SARS-CoV-2 does not appear to be lethal as SARS-CoV, it has rapidly spread worldwide according to a World Health Organization situation report. The rapid spread of SARS-CoV-2 and its ability to cause death particular in older individuals, or individuals with underlying conditions, has created an urgency for the need of antiviral therapeutics and vaccines for use against the virus ("CDC People at Risk for Serious Illness from COVID-19," CDC website).

Antiviral chemotherapeutics that target SARS-CoV-2 virus are not yet available and the possibility of rapid emergence of drug-resistant strains is likely. Consequently, there is a burgeoning need to identify new anti-SARS-CoV-2 therapeutics, particularly those that target host gene products required for virus replication, to reduce the likelihood of drug resistance.

While there has been significant focus on vaccinations to eliminate or control SARS-CoV-2 infection. However, the effectiveness of the vaccines is impacted by limited manufacturing capacity, cold chain storage requirements, viral infection breakthroughs, limited efficacy data, viral shedding from assymptomic immunized subjects, delays in and/or phased vaccine rollout, fear of the vaccines, on-going mutation/evolution of the virus, likelihood of COVID-19 to remain endemic worldwide. Accordingly, there remains a strong need for therapeutic options for the treatment and prevention of SARS-CoV-2 infection and COVID-19 disease.

Thus, it is an object of the invention to provide compositions and methods of treating infections caused by coronaviruses, particularly coronaviruses that cause severe acute respiratory syndrome, including, but limited to SARS-CoV-2.

### SUMMARY OF THE INVENTION

Compositions and methods of treating a subject for a SARS-CoV-2 infection are provided. The methods typically include administering the subject an effective amount of probenecid, a metabolite or analog thereof, or a pharmaceutically acceptable salt thereof. The amount of probenecid, metabolite or analog thereof, or a pharmaceutically acceptable salt thereof can be effective to, for example, reduce viral replication, reduce one or more symptoms of a disease, disorder, or illness associated with virus, or a combination thereof. Symptoms include, but are not limited to, fever, congestion in the nasal sinuses and/or lungs, runny or stuffy nose, cough, sneezing, sore throat, body aches, fatigue, shortness of breath, chest tightness, wheezing when exhaling, chills, muscle aches, headache, diarrhea, tiredness, nausea, vomiting, and combinations thereof. The subject can be, for example, a mammal or a bird. In preferred embodiments, the subject is a human.

The subject can be symptomatic or asymptomatic. In some embodiments, the subject has been, or will be, exposed to the virus. In some embodiments, treatment begins 1, 2, 3, 4, 5, or more hours, days, or weeks prior to or after exposure to the virus. In some embodiments, the subject has not been exposed to the virus. In some embodiments, the subject anticipates being exposed to the virus. Thus, preventative and prophylactic methods are also provided, and are included in the term 'treatment'.

The virus is SARS-CoV-2.

In some embodiments, the virus is a severe acute respiratory syndrome coronavirus 2 (SARS-CoV-2) having a genome encoded by a nucleic acid sequence having at least 85%, 86%, 87%, 88%, 89%, 90%, 91%, 92%, 93%, 94%, 95%, 96%, 97%, 98%, 99%, or 100% sequence identity SEQ ID NO:1 or 2.

In some embodiments, the SARS-CoV-2 includes a Spike protein having at least 70%, 75%, 80%, 85%, 86%, 87%, 88%, 89%, 90%, 91%, 92%, 93%, 94%, 95%, 96%, 97%, 98%, 99%, or 100% sequence identity to SEQ ID NO:5. For example, the SAR-CoV-2 can have one or more mutations relative to SEQ ID NO:5. In some embodiments, the mutations are selected from H69, optionally deletion thereof; V70, optionally deletion thereof; G142, optionally G142D; K417, optionally K417N or K417T; E484, optionally E484K; F486, optionally F486L; and N501 mutation, optionally N501Y or N501T, and combinations thereof. The SARS-CoV-2 can be from, for example, the B.1.1.7, B.1.351, P.1, B.1.1.207, B.1.429, B.1.427, or B.1.525 lineage.

In some embodiments, the subject has a disease or disorder associated with the virus. For example, in embodiments, a subject exposed or infected with SARS-CoV-2 and optionally has Coronavirus Disease 2019 (COVID-19). In some embodiments, the subject is not infected with influenza.

In some embodiments, a viral infection is detected or diagnosed in a subject prior to, during, or after treatment. Detection and diagnosis of viral infection may include, but is not limited to, PCR tests designed to detect viral RNA, and serological and immunodiagnostic tests designed to detect antibodies against the virus. An exemplary test for SARS-CoV-2 infection/COVID-19 is the iAMP COVID-19 Detection Kit, which is a real-time fluorescent isothermal assay for use on raw samples without RNA extraction.

In some embodiments, the subject has been in close contact with a person that has tested positive for the virus or has COVID-19. Such a person may or may not be exhibiting one or more symptoms of an infection. In some embodiments, the subject of the treatment is identified by contact tracing as having been exposed to the virus, or one or more persons infected therewith.

The probenecid, metabolite or analog thereof, or pharmaceutically acceptable salt thereof is typically administered in a pharmaceutical composition including a pharmaceutically acceptable carrier and/or excipient. Thus, pharmaceutical compositions are also provided. Dosage forms are also provided and include, but not limited to 500 mg tablets of probenecid, a metabolite or analog thereof, or pharmaceutically acceptable salt thereof. In some embodiments, the subject is administered a 10 mg - 1,000 mg or, 50 mg - 500 mg dose of probenecid, metabolite or analog thereof, or pharmaceutically acceptable salt thereof 1, 2, 3, 4, or 5 times per day. In some embodiments, the dosage regimen is a pulse dosage regimen that includes 1, 2, 3, or more large (1,000 mg or more) bolus doses in close proximity (e.g., at most 5, 10, 15, 30, 45, or 60 minutes, or 1, 2, 3, 4, 5, 6, or more hours apart). In some embodiments, the bolus doses are followed by a drug administration holiday (e.g. at least 12 hours, or 1, 2, 3, 4, 5, or more days), optionally until the drug level in the subject's serum drops to zero or near zero (e.g., no more than 1%, 5%, 10%, or 20% of the peak serum level).

The probenecid, a metabolite or analog thereof, or a pharmaceutically acceptable salt thereof can be administered systemically or locally. Exemplary routes of administration include, but are not limited to, oral, parenteral, topical or mucosal. In some embodiments, the composition is administered to lungs (e.g., pulmonary administration) by oral inhalation or intranasal administration. In some embodiments, the composition is administered intranasally to the nasal mucosa.

### BRIEF DESCRIPTION OF THE DRAWINGS

**Figure 1** is a bar graph showing (left-to-right) the effect of probenecid (5 µM, 2.5 µM, 1 µM, or 0.1 µM) compared to controls (DMSO (infected), DMSO (only)) on viral replication using a plaque reduction assay.
**Figure 2** is a bar graph showing (left-to-right) the effect of probenecid pre-treatment (5 µM, 2.5 µM, 1 µM, 0.1 µM 0.01 µM, 0.001 µM, or 0.0001 µM) compared to controls (DMSO (infected), DMSO (only)) on viral replication using a plaque reduction assay.
**Figure 3** is a bar graph showing (left-to-right) the effect of probenecid post-treatment (5 µM, 2.5 µM, 1 µM, 0.1 µM 0.01 µM, 0.001 µM, or 0.0001 µM) compared to controls (DMSO (infected), DMSO (only)) on viral replication using a plaque reduction assay.
**Figure 4** is a flow diagram showing an assay for testing the prophylactic (pre-infection) and therapeutic (post-infection) effect of probenecid on SARS-CoV-2 in hamsters *in vivo.*
**Figure 5** is a line graph showing the weight of hamsters in control and experimental groups prior to and subsequent to treatment with probenecid and/or infection.
**Figure 6** is a bar graph showing TCID₅₀ (Log TCID₅₀/ml) from lungs of hamster treated with probenecid.
**Figure 7** is a bar graph showing the results of a plaque assay (Log PFU/ml) from lungs of hamster lungs treated with probenecid.
**Figure 8A** is a bar graph showing the Body Mass Index (BMI) of human subjects enrolled in an open label, ten patients, twenty-eight (28) day, Investigator Initiated Study (IIS) ("clinical trial") testing the effect of probenecid on subjects with mild to moderate SARS-CoV-2 infection. **Figure 8B** is a bar graph showing the age (years) of subjects enrolled in the clinical trial.
**Figure 9** is a bar graph showing day until a negative SARS-CoV-2 test result in subject of the clinical trial.
**Figure 10** is a bar graph showing improvement in symptoms in subjects during the clinical trial.
**Figure 11** is a graph showing changes in body temperature in subjects during the clinical trial.
**Figure 12** is a graph showing changes in high sensitivity C ractive protein (hs-CRP) in subjects during the clinical trial.
**Figure 13** is a graph showing changes in d-dimer in subjects during the clinical trial.
**Figure 14** is a graph showing changes in fibrinogen in subjects during the clinical trial.
**Figure 15** is a graph showing changes in lactate dehydrogenase (LDH) in subjects during the clinical trial.

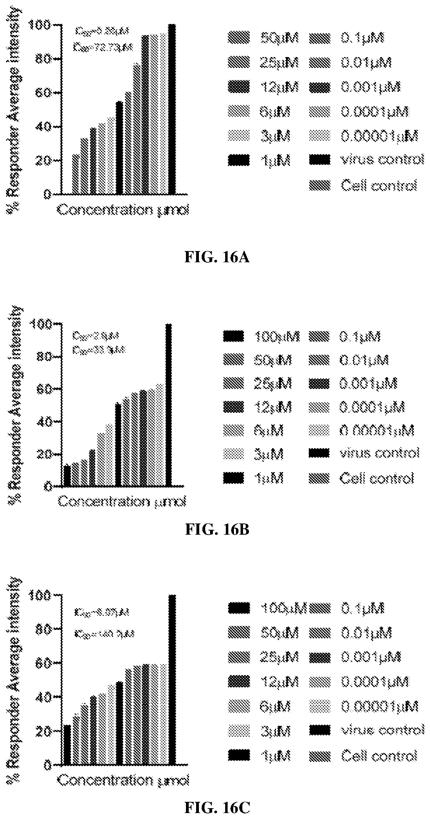

**Figures 16A-16C** are bar graphs showing % responder average intensity of SARS-CoV-2-infected Normal Human Bronchial Epithelial (NHBE) cells prophylactically treated with (from left-to-right) 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control (**16A**); 100 µM, 50 µM, 25 µM, 12 µM, 6 µM, 3 µM, 1 µM, 0.1 µM, 0.01 µM, 0.001 µM, 0.0001 µM, or 0.00001 µM of probenecid, virus control, or cell control (**16B**); or 0.1 µM probenecid + 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control (**16C**).

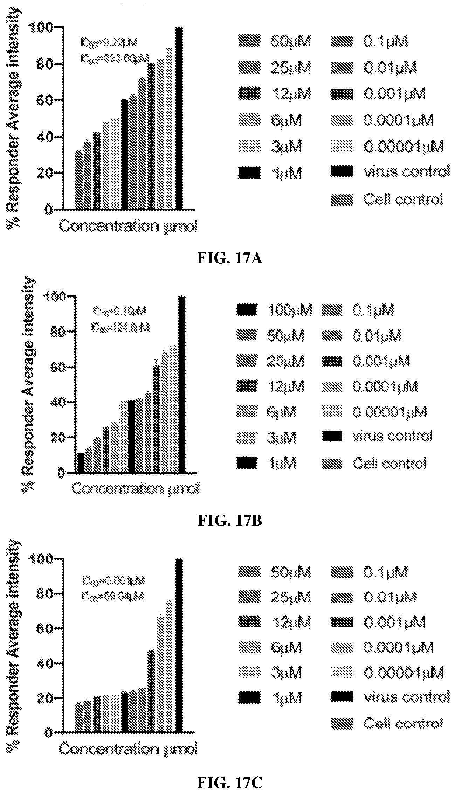

**Figures 17A-17C** are bar graphs showing % responder average intensity of SARS-CoV-2-infected Normal Human Bronchial Epithelial (NHBE) cells treated at the time of infection with (from left-to-right) 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control (**17A**); 100 µM, 50 µM, 25 µM, 12 µM, 6 µM, 3 µM, 1 µM, 0.1 µM, 0.01 µM, 0.001 µM, 0.0001 µM, or 0.00001 µM of probenecid, virus control, or cell control (**17B**); or 0.1 µM probenecid + 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control (**17C**).

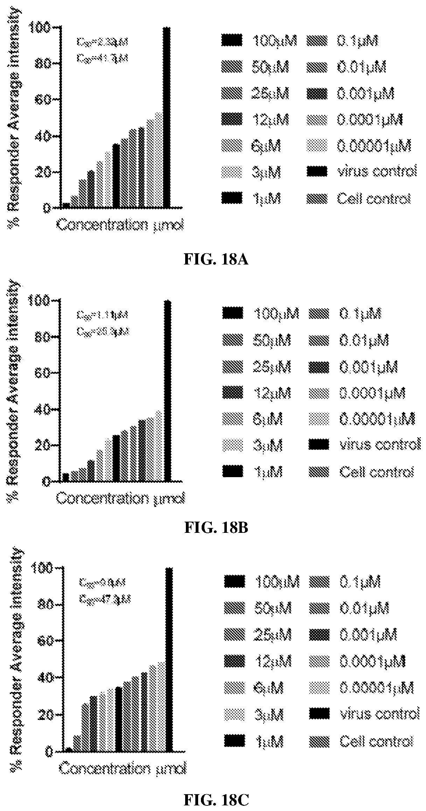

**Figures 18A-18C** are bar graphs showing % responder average intensity of SARS-CoV-2-infected Vero cells prophylactically treated with (from left-to-right) 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control (**18A**); 100 µM, 50 µM, 25 µM, 12 µM, 6 µM, 3 µM, 1 µM, 0.1 µM, 0.01 µM, 0.001 µM, 0.0001 µM, or 0.00001 µM of probenecid, virus control, or cell control (**18B**); or 0.1 µM probenecid + 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control (**18C**).

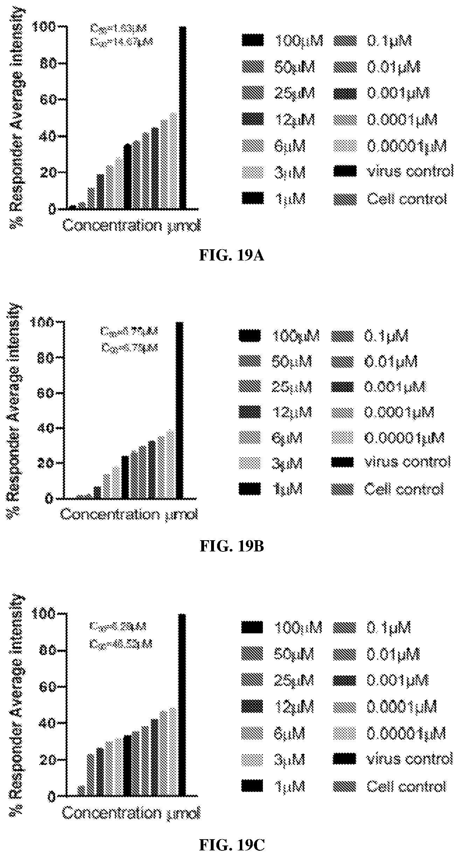

**Figures 19A-19C** are bar graphs showing % responder average intensity of SARS-CoV-2-infected Vero cells treated at the time of infection with (from left-to-right) 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control (**19A**); 100 µM, 50 µM, 25 µM, 12 µM, 6 µM, 3 µM, 1 µM, 0.1 µM, 0.01 µM, 0.001 µM, 0.0001 µM, or 0.00001 µM of probenecid, virus control, or cell control (**19B**); or 0.1 µM probenecid + 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control (**19C**).

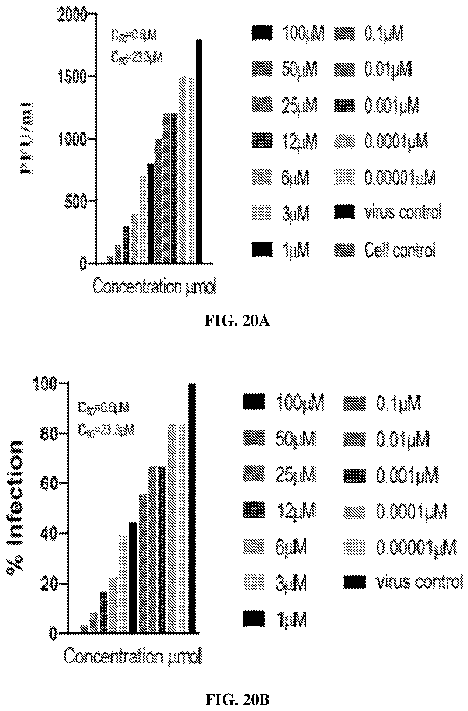

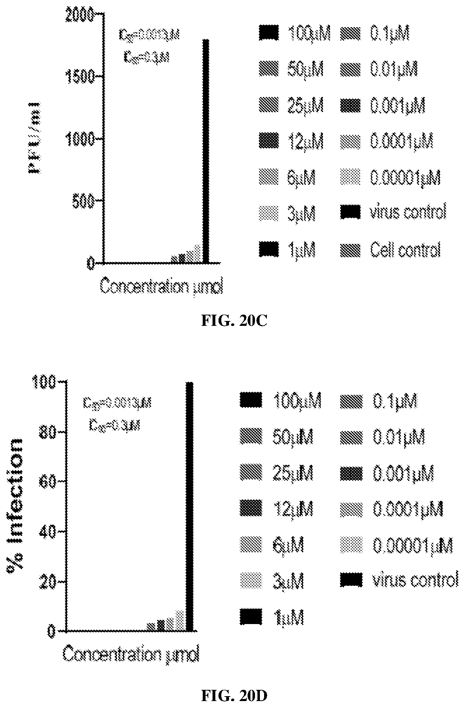

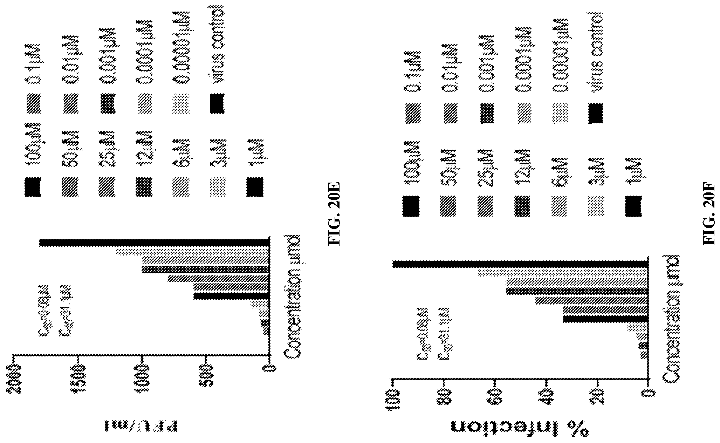

**Figure 20A** is a bar graph showing PFU/ml of SARS-CoV-2-infected Normal Human Bronchial Epithelial (NHBE) cells treated at the time of infection with (from left-to-right) 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control. **Figure 20B** is a bar graph showing % Infection of SARS-CoV-2-infected Normal Human Bronchial Epithelial (NHBE) cells treated at the time of infection with (from left-to-right) 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, or virus control. **Figure 20C** is a bar graph showing PFU/ml of SARS-CoV-2-infected Normal Human Bronchial Epithelial (NHBE) cells treated at the time of infection with (from left-to-right) 100 µM, 50 µM, 25 µM, 12 µM, 6 µM, 3 µM, 1 µM, 0.1 µM, 0.01 µM, 0.001 µM, 0.0001 µM, or 0.00001 µM of probenecid, virus control, or cell control. **Figure 20D** is a bar graph showing % Infection of SARS-CoV-2-infected Normal Human Bronchial Epithelial (NHBE) cells treated at the time of infection with (from left-to-right) 100 µM, 50 µM, 25 µM, 12 µM, 6 µM, 3 µM, 1 µM, 0.1 µM, 0.01 µM, 0.001 µM, 0.0001 µM, or 0.00001 µM of probenecid, or virus control. **Figure 20E** is a bar graph showing PFU/ml of SARS-CoV-2-infected Normal Human Bronchial Epithelial (NHBE) cells treated at the time of infection with (from left-to-right) 0.1 µM probenecid + 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, virus control, or cell control. **Figure 20F** is a bar graph showing % Infection of SARS-CoV-2-infected Normal Human Bronchial Epithelial (NHBE) cells treated at the time of infection with (from left-to-right) 0.1 µM probenecid + 100 µM, 50 µM, 25 µM, 12 µM, 6 µM, 3 µM, 1 µM, 0.1 µM, 0.01 µM, 0.001 µM, 0.0001 µM, or 0.00001 µM of remdesivir, or virus control.
**Figure 21** is bar graphs showing the number of plaques of SARS-CoV-2 B.1.1.7-infected Vero cells treated at the time of infection with (from left-to-right) 100 µM, 50 µM, 25 µM, 12 µM, 6 µM, 3 µM, 1 µM, 0.1 µM, 0.01 µM, 0.001 µM, 0.0001 µM, or 0.00001 µM of probenecid, virus control, or cell control.

### DETAILED DESCRIPTION OF THE INVENTION

### I. Definitions

As used herein, the terms "individual", "host", "subject", and "patient" are used interchangeably herein, and refer animals, particularly birds and mammals, including, but not limited to, primates such as humans, bats, rodents, such as mice and rats, and other laboratory animals.

As used herein the term "effective amount" or "therapeutically effective amount" means a dosage sufficient to treat, inhibit, or alleviate one or more symptoms of a disease state being treated or to otherwise provide a desired pharmacologic and/or physiologic effect. The precise dosage will vary according to a variety of factors such as subject-dependent variables (e.g., age, immune system health, etc.), the disease, and the treatment being administered.

As used herein, the term "carrier" or "excipient" refers to an organic or inorganic ingredient, natural or synthetic inactive ingredient in a formulation, with which one or more active ingredients are combined.

As used herein, the term "pharmaceutically acceptable" means a nontoxic material that does not interfere with the effectiveness of the biological activity of the active ingredients.

As used herein, the term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approx. +/- 10%; in other forms the values may range in value either above or below the stated value in a range of approx. +/- 5%; in other forms the values may range in value either above or below the stated value in a range of approx. +/- 2%; in other forms the values may range in value either above or below the stated value in a range of approx. +/- 1%. The preceding ranges are intended to be made clear by context, and no further limitation is implied.

Disclosed are materials, compositions, and components that can be used for, can be used in conjunction with, can be used in preparation for, or are products of the disclosed method and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a ligand is disclosed and discussed and a number of modifications that can be made to a number of molecules including the ligand are discussed, each and every combination and permutation of ligand and the modifications that are possible are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. Further, each of the materials, compositions, components, etc. contemplated and disclosed as above can also be specifically and independently included or excluded from any group, subgroup, list, set, etc. of such materials.

These concepts apply to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

All methods described herein can be performed in any suitable order unless otherwise indicated or otherwise clearly contradicted by context. The use of any and all examples, or exemplary language (e.g., "such as") provided herein, is intended merely to better illuminate the embodiments and does not pose a limitation on the scope of the embodiments unless otherwise claimed. No language in the specification should be construed as indicating any non-claimed element as essential to the practice of the invention.

### II. Compositions

### A. Probenecid, Metabolites, Analogs, and Pharmaceutically Acceptable Salts Thereof

The disclosed methods include administering a subject in need thereof an effective amount of probenecid, a metabolite or analog thereof, or a pharmaceutically acceptable salt thereof, including, but not limited to a sodium salt thereof.

Probenecid (4-[(dipropylamino) sulfony1] benzoic acid (CAS No. 57-66-9)) has the structure: 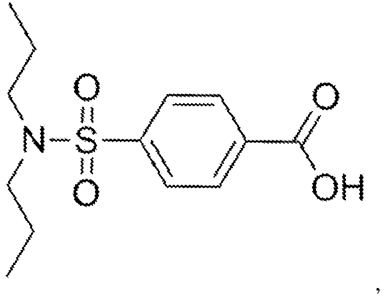 and has been sold under the brand names BENEMID^{®} and PROBALAN^{®}.

Probenecid is a highly lipid soluble benzoic acid derivative with an excellent safety profile that was developed in the 1950's to decrease the renal tubular excretion of penicillin. Probenecid, USP is a white or nearly white, fine, crystalline powder. Probenecid is soluble in dilute alkali, in alcohol, in chloroform, and in acetone; it is practically insoluble in water and in dilute acids. It has a half life of 6-12 hours. See also Drugbank Accession Number DB01032 (APRD00167), and PubChem CID 4911.

Metabolites and analogs of probenecid are known, see, for example, Guarino, et al., "Mass spectral identification of probenecid metabolites in rat bile," Eur. J. Pharmacol., 8, 244-252 (1969), Perel, et al., "Identification and renal excretion of probenecid metabolites in man," Life Sciences, 9, 23, 1337-1343 (1970), Perel, et al., "Studies of the renal excretion of probenecid acyl glucuronide in man," Eur. J. Clin. Pharmacol, 3, 106-112 (1971), Dayton and Perel, "The metabolism of probenecid in man,". N. Y. Acad. Sci., 179, 399-402 (1971), Dayton, et al., "The effect of probenecid, phenylbutazone and their analogues on the excretion of L-ascorbic acid in rats," J. Med. Chem. 9, 941-944 (1966), and Israili, et al., "Metabolites of probenecid. Chemical, physical, and pharmacological studies," J. Med. Chem., 15, 7, 709-713 (1972).

In some embodiments, the metabolite is a glucuronide derivative of probenecid such as acyl glucuronide or a β-ether glucuronide.

Further probenecid metabolites and analogs that may be used in the invention are:
d1-p-(N-propyl-N-2-hydroxypropylsulfamoyl)benzoic acid,
p-(N-Propyl-N-3-hydroxy-propyl-sulfamoyl)-benzoic acid,
p-(N-propyl-N-3-propionitrilosulfamoyl)benzoic acid,
p-(N-propyl-N-2-carboxy--ethyl-sulfamoyl)-benzoic acid,
p-(N-propylsulfamoyl)benzoic acid,
p-(N,N-penta-methylene-sulfamoyl)-benzoic acid (piperidyl analog),
p-(N-propyl-N-2-propenyl-sulfamoyl)-benzoic acid,
p-(N-propyl-N-2-oxopropylsulfamoyl)benzoic acid,
p-sulfamoylbenzoic acid,
p-(N-methylsulfamoyl)benzoic acid,
p-(N-ethylsulfamoyl)benzoic acid,
p-(N-hexylsulfamoyl)benzoic acid,
p-(N-cyclohexylsulfamoyl)benzoic acid,
p-(N-dimethylsulfamoyl)benzoic acid,
p-(N-methyl,N-ethylsulfamoyl)benzoic acid,
p-(N,N-diethylsulfamoyl)benzoic acid,
p-(N-ethyl,N-propylsulfamoyl)benzoic acid,
p-(N,N-diiso-propyl-sulfamoyl)benzoic acid,
p-(N,N-dibutylsulfamoyl)benzoic acid,
o-hydroxy-p-(N-dipropyl-sulfamoyl)benzoic acid,
o-methoxy-p-(N-dipropylsulfamoyl)benzoic acid,
o-nitro-p-(N,N-dipropylsulfamoyl)benzoic acid,
m-nitro-p-(N,N-dipropyl-sulfamoyl)benzoic acid or
m-methyl-p-(N,N-dipropylsulfamoyl)benzoic acid.

Typically the metabolite or analog can on its own, or upon further metabolism thereof by a subject, treat a coronavirus when administered in an effective amount as discussed herein. For example, in some embodiments, the metabolite or analog can on its own, or upon further metabolism thereof by a subject, reduce viral replication.

### B. Formulations

Probenecid, metabolites and analogs thereof, and pharmaceutically acceptable salts thereof can be formulated in a pharmaceutical composition. Pharmaceutical compositions can be for administration by parenteral (intramuscular, intraperitoneal, intravenous (IV) or subcutaneous injection), enteral, transdermal (either passively or using iontophoresis or electroporation), or transmucosal (nasal, pulmonary, vaginal, rectal, or sublingual) routes of administration or using bioerodible inserts and can be formulated in dosage forms appropriate for each route of administration.

The compositions can be administered systemically.

The compositions can be formulated for immediate release, extended release, or modified release. A delayed release dosage form is one that releases a drug (or drugs) at a time other than promptly after administration. An extended release dosage form is one that allows at least a twofold reduction in dosing frequency as compared to that drug presented as a conventional dosage form (e.g. as a solution or prompt drug-releasing, conventional solid dosage form). A modified release dosage form is one for which the drug release characteristics of time course and/or location are chosen to accomplish therapeutic or convenience objectives not offered by conventional dosage forms such as solutions, ointments, or promptly dissolving dosage forms. Delayed release and extended release dosage forms and their combinations are types of modified release dosage forms.

Formulations are prepared using a pharmaceutically acceptable "carrier" composed of materials that are considered safe and effective and may be administered to an individual without causing undesirable biological side effects or unwanted interactions. The "carrier" is all components present in the pharmaceutical formulation other than the active ingredient or ingredients. The term "carrier" includes, but is not limited to, diluents, binders, lubricants, disintegrators, fillers, and coating compositions.

"Carrier" also includes all components of the coating composition which may include plasticizers, pigments, colorants, stabilizing agents, and glidants. The delayed release dosage formulations may be prepared as described in references such as "Pharmaceutical dosage forms: tablets", eds. Liberman et. al. (New York, Marcel Dekker, Inc., 1989), "Remington - The science and practice of pharmacy", 21st ed., Lippincott Williams & Wilkins, Baltimore, MD, 2006, and "Ansel's Pharmaceutical dosage forms and drug delivery systems", 11th Edition, Loyd Allen., (Media, PA: Williams and Wilkins, 2017) which provides information on carriers, materials, equipment and process for preparing tablets and capsules and delayed release dosage forms of tablets, capsules, and granules.

The compound can be administered to a subject with or without the aid of a delivery vehicle. Appropriate delivery vehicles for the compounds are known in the art and can be selected to suit the particular active agent. For example, in some embodiments, the active agent(s) is incorporated into or encapsulated by, or bound to, a nanoparticle, microparticle, micelle, synthetic lipoprotein particle, or carbon nanotube. For example, the compositions can be incorporated into a vehicle such as polymeric microparticles which provide controlled release of the active agent(s). In some embodiments, release of the drug(s) is controlled by diffusion of the active agent(s) out of the microparticles and/or degradation of the polymeric particles by hydrolysis and/or enzymatic degradation.

Suitable polymers include ethylcellulose and other natural or synthetic cellulose derivatives. Polymers which are slowly soluble and form a gel in an aqueous environment, such as hydroxypropyl methylcellulose or polyethylene oxide, may also be suitable as materials for drug containing microparticles or particles. Other polymers include, but are not limited to, polyanhydrides, poly (ester anhydrides), polyhydroxy acids, such as polylactide (PLA), polyglycolide (PGA), poly(lactide-co-glycolide) (PLGA), poly-3-hydroxybut rate (PHB) and copolymers thereof, poly-4-hydroxybutyrate (P4HB) and copolymers thereof, polycaprolactone and copolymers thereof, and combinations thereof. In some embodiments, both agents are incorporated into the same particles and are formulated for release at different times and/or over different time periods. For example, in some embodiments, one of the agents is released entirely from the particles before release of the second agent begins. In other embodiments, release of the first agent begins followed by release of the second agent before all of the first agent is released. In still other embodiments, both agents are released at the same time over the same period of time or over different periods of time.

### 1. Oral Immediate Release Formulations

Suitable oral dosage forms include tablets, capsules, solutions, suspensions, syrups, and lozenges. Thus, the composition can be formulated as a solid or liquid. Tablets can be made using compression or molding techniques well known in the art. Gelatin or non-gelatin capsules can be prepared as hard or soft capsule shells, which can encapsulate liquid, solid, and semi-solid fill materials, using techniques well known in the art.

Examples of suitable coating materials include, but are not limited to, cellulose polymers such as cellulose acetate phthalate, hydroxypropyl cellulose, hydroxypropyl methylcellulose, hydroxypropyl methylcellulose phthalate and hydroxypropyl methylcellulose acetate succinate; polyvinyl acetate phthalate, acrylic acid polymers and copolymers, and methacrylic resins that are commercially available under the trade name Eudragit^{®} (Roth Pharma, Westerstadt, Germany), Zein, shellac, and polysaccharides.

Additionally, the coating material may contain conventional carriers such as plasticizers, pigments, colorants, glidants, stabilization agents, pore formers and surfactants.

Optional pharmaceutically acceptable excipients present in the drug-containing tablets, beads, granules or particles include, but are not limited to, diluents, binders, lubricants, disintegrants, colorants, stabilizers, and surfactants. Diluents, also termed "fillers," are typically necessary to increase the bulk of a solid dosage form so that a practical size is provided for compression of tablets or formation of beads and granules. Suitable diluents include, but are not limited to, dicalcium phosphate dihydrate, calcium sulfate, lactose, sucrose, mannitol, sorbitol, cellulose, microcrystalline cellulose, kaolin, sodium chloride, dry starch, hydrolyzed starches, pregelatinized starch, silicone dioxide, titanium oxide, magnesium aluminum silicate and powder sugar.

Binders are used to impart cohesive qualities to a solid dosage formulation, and thus ensure that a tablet or bead or granule remains intact after the formation of the dosage forms. Suitable binder materials include, but are not limited to, starch, pregelatinized starch, gelatin, sugars (including sucrose, glucose, dextrose, lactose and sorbitol), polyethylene glycol, waxes, natural and synthetic gums such as acacia, tragacanth, sodium alginate, cellulose, including hydroxypropylmethylcellulose, hydroxypropylcellulose, ethylcellulose, and veegum, and synthetic polymers such as acrylic acid and methacrylic acid copolymers, methacrylic acid copolymers, methyl methacrylate copolymers, aminoalkyl methacrylate copolymers, polyacrylic acid/polymethacrylic acid and polyvinylpyrrolidone.

Lubricants are used to facilitate tablet manufacture. Examples of suitable lubricants include, but are not limited to, magnesium stearate, calcium stearate, stearic acid, glycerol behenate, polyethylene glycol, talc, and mineral oil.

Disintegrants are used to facilitate dosage form disintegration or "breakup" after administration, and generally include, but are not limited to, starch, sodium starch glycolate, sodium carboxymethyl starch, sodium carboxymethylcellulose, hydroxypropyl cellulose, pregelatinized starch, clays, cellulose, alginine, gums or cross linked polymers, such as crosslinked PVP (Polyplasdone XL from GAF Chemical Corp).

Stabilizers are used to inhibit or retard drug decomposition reactions which include, by way of example, oxidative reactions.

Surfactants may be anionic, cationic, amphoteric or nonionic surface active agents. Suitable anionic surfactants include, but are not limited to, those containing carboxylate, sulfonate and sulfate ions. Examples of anionic surfactants include sodium, potassium, ammonium of long chain alkyl sulfonates and alkyl aryl sulfonates such as sodium dodecylbenzene sulfonate; dialkyl sodium sulfosuccinates, such as sodium dodecylbenzene sulfonate; dialkyl sodium sulfosuccinates, such as sodium bis-(2-ethylthioxyl)-sulfosuccinate; and alkyl sulfates such as sodium lauryl sulfate. Cationic surfactants include, but are not limited to, quaternary ammonium compounds such as benzalkonium chloride, benzethonium chloride, cetrimonium bromide, stearyl dimethylbenzyl ammonium chloride, polyoxyethylene and coconut amine. Examples of nonionic surfactants include ethylene glycol monostearate, propylene glycol myristate, glyceryl monostearate, glyceryl stearate, polyglyceryl-4-oleate, sorbitan acylate, sucrose acylate, PEG-150 laurate, PEG-400 monolaurate, polyoxyethylene monolaurate, polysorbates, polyoxyethylene octylphenylether, PEG-1000 cetyl ether, polyoxyethylene tridecyl ether, polypropylene glycol butyl ether, POLOXAMER^{®} 401, stearoyl monoisopropanolamide, and polyoxyethylene hydrogenated tallow amide. Examples of amphoteric surfactants include sodium N-dodecyl-.beta.-alanine, sodium N-lauryl-.beta.-iminodipropionate, myristoamphoacetate, lauryl betaine and lauryl sulfobetaine.

If desired, the tablets, beads granules or particles may also contain minor amount of nontoxic auxiliary substances such as wetting or emulsifying agents, dyes, pH buffering agents, and preservatives.

### 2. Extended release dosage forms

The extended release formulations are generally prepared as diffusion or osmotic systems, for example, as described in "Remington - The science and practice of pharmacy" (21st ed., Lippincott Williams & Wilkins, Baltimore, MD, 2006). A diffusion system typically consists of two types of devices, reservoir and matrix, and is well known and described in the art. The matrix devices are generally prepared by compressing the drug with a slowly dissolving polymer carrier into a tablet form. The three major types of materials used in the preparation of matrix devices are insoluble plastics, hydrophilic polymers, and fatty compounds. Plastic matrices include, but not limited to, methyl acrylate-methyl methacrylate, polyvinyl chloride, and polyethylene. Hydrophilic polymers include, but are not limited to, methylcellulose, hydroxypropylcellulose, hydroxypropylmethylcellulose, sodium carboxymethylcellulose, and carbopol 934, polyethylene oxides. Fatty compounds include, but are not limited to, various waxes such as carnauba wax and glyceryl tristearate.

Alternatively, extended release formulations can be prepared using osmotic systems or by applying a semi-permeable coating to the dosage form. In the latter case, the desired drug release profile can be achieved by combining low permeable and high permeable coating materials in suitable proportion.

The devices with different drug release mechanisms described above could be combined in a final dosage form comprising single or multiple units. Examples of multiple units include multilayer tablets, capsules containing tablets, beads, granules, etc.

An immediate release portion can be added to the extended release system by means of either applying an immediate release layer on top of the extended release core using coating or compression process or in a multiple unit system such as a capsule containing extended and immediate release beads.

Extended release tablets containing hydrophilic polymers are prepared by techniques commonly known in the art such as direct compression, wet granulation, or dry granulation processes. Their formulations usually incorporate polymers, diluents, binders, and lubricants as well as the active pharmaceutical ingredient. The usual diluents include inert powdered substances such as any of many different kinds of starch, powdered cellulose, especially crystalline and microcrystalline cellulose, sugars such as fructose, mannitol and sucrose, grain flours and similar edible powders. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders include substances such as starch, gelatin and sugars such as lactose, fructose, and glucose. Natural and synthetic gums, including acacia, alginates, methylcellulose, and polyvinylpyrrolidine can also be used. Polyethylene glycol, hydrophilic polymers, ethylcellulose and waxes can also serve as binders. A lubricant is necessary in a tablet formulation to prevent the tablet and punches from sticking in the die. The lubricant is chosen from such slippery solids as talc, magnesium and calcium stearate, stearic acid and hydrogenated vegetable oils.

Extended release tablets containing wax materials are generally prepared using methods known in the art such as a direct blend method, a congealing method, and an aqueous dispersion method. In a congealing method, the drug is mixed with a wax material and either spray- congealed or congealed and screened and processed.

### 3. Delayed release dosage forms

Delayed release formulations are created by coating a solid dosage form with a film of a polymer that is insoluble in the acid environment of the stomach, and soluble in the neutral environment of small intestines.

The delayed release dosage units can be prepared, for example, by coating a drug or a drug-containing composition with a selected coating material. The drug-containing composition may be, e.g., a tablet for incorporation into a capsule, a tablet for use as an inner core in a "coated core" dosage form, or a plurality of drug-containing beads, particles or granules, for incorporation into either a tablet or capsule. Preferred coating materials include bioerodible, gradually hydrolyzable, gradually water-soluble, and/or enzymatically degradable polymers, and may be conventional "enteric" polymers. Enteric polymers, as will be appreciated by those skilled in the art, become soluble in the higher pH environment of the lower gastrointestinal tract or slowly erode as the dosage form passes through the gastrointestinal tract, while enzymatically degradable polymers are degraded by bacterial enzymes present in the lower gastrointestinal tract, particularly in the colon. Suitable coating materials for effecting delayed release include, but are not limited to, cellulosic polymers such as hydroxypropyl cellulose, hydroxyethyl cellulose, hydroxymethyl cellulose, hydroxypropyl methyl cellulose, hydroxypropyl methyl cellulose acetate succinate, hydroxypropylmethyl cellulose phthalate, methylcellulose, ethyl cellulose, cellulose acetate, cellulose acetate phthalate, cellulose acetate trimellitate and carboxymethylcellulose sodium; acrylic acid polymers and copolymers, preferably formed from acrylic acid, methacrylic acid, methyl acrylate, ethyl acrylate, methyl methacrylate and/or ethyl methacrylate, and other methacrylic resins that are commercially available under the tradename EUDRAGIT^{®}. (Rohm Pharma; Westerstadt, Germany), including EUDRAGIT^{®}. L30D-55 and L100-55 (soluble at pH 5.5 and above), EUDRAGIT^{®}. L-100 (soluble at pH 6.0 and above), EUDRAGIT^{®}. S (soluble at pH 7.0 and above, as a result of a higher degree of esterification), and EUDRAGITS^{®}. NE, RL and RS (water-insoluble polymers having different degrees of permeability and expandability); vinyl polymers and copolymers such as polyvinyl pyrrolidone, vinyl acetate, vinylacetate phthalate, vinylacetate crotonic acid copolymer, and ethylene-vinyl acetate copolymer; enzymatically degradable polymers such as azo polymers, pectin, chitosan, amylose and guar gum; zein and shellac. Combinations of different coating materials may also be used. Multi-layer coatings using different polymers may also be applied.

The preferred coating weights for particular coating materials may be readily determined by those skilled in the art by evaluating individual release profiles for tablets, beads and granules prepared with different quantities of various coating materials. It is the combination of materials, method and form of application that produce the desired release characteristics, which one can determine only from the clinical studies.

The coating composition may include conventional additives, such as plasticizers, pigments, colorants, stabilizing agents, glidants, etc. A plasticizer is normally present to reduce the fragility of the coating, and will generally represent about 10 wt. % to 50 wt. % relative to the dry weight of the polymer. Examples of typical plasticizers include polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil and acetylated monoglycerides. A stabilizing agent is preferably used to stabilize particles in the dispersion. Typical stabilizing agents are nonionic emulsifiers such as sorbitan esters, polysorbates and polyvinylpyrrolidone. Glidants are recommended to reduce sticking effects during film formation and drying, and will generally represent approximately 25 wt. % to 100 wt. % of the polymer weight in the coating solution. One effective glidant is talc. Other glidants such as magnesium stearate and glycerol monostearates may also be used. Pigments such as titanium dioxide may also be used. Small quantities of an anti-foaming agent, such as a silicone (e.g., simethicone), may also be added to the coating composition.

### Methods of manufacturing

As will be appreciated by those skilled in the art and as described in the pertinent texts and literature, a number of methods are available for preparing drug-containing tablets, beads, granules or particles that provide a variety of drug release profiles. Such methods include, but are not limited to, the following: coating a drug or drug-containing composition with an appropriate coating material, typically although not necessarily incorporating a polymeric material, increasing drug particle size, placing the drug within a matrix, and forming complexes of the drug with a suitable complexing agent.

The delayed release dosage units may be coated with the delayed release polymer coating using conventional techniques, e.g., using a conventional coating pan, an airless spray technique, fluidized bed coating equipment (with or without a Wurster insert). For detailed information concerning materials, equipment and processes for preparing tablets and delayed release dosage forms, see Pharmaceutical Dosage Forms: Tablets, eds. Lieberman et al. (New York: Marcel Dekker, Inc., 1989), and Ansel's Pharmaceutical Dosage Forms and Drug Delivery Systems, 11th Ed. (Media, PA: Williams & Wilkins, 2017).

A preferred method for preparing extended release tablets is by compressing a drug-containing blend, e.g., blend of granules, prepared using a direct blend, wet-granulation, or dry-granulation process. Extended release tablets may also be molded rather than compressed, starting with a moist material containing a suitable water-soluble lubricant. However, tablets are preferably manufactured using compression rather than molding. A preferred method for forming extended release drug-containing blend is to mix drug particles directly with one or more excipients such as diluents (or fillers), binders, disintegrants, lubricants, glidants, and colorants. As an alternative to direct blending, a drug-containing blend may be prepared by using wet-granulation or dry-granulation processes. Beads containing the active agent may also be prepared by any one of a number of conventional techniques, typically starting from a fluid dispersion. For example, a typical method for preparing drug-containing beads involves dispersing or dissolving the active agent in a coating suspension or solution containing pharmaceutical excipients such as polyvinylpyrrolidone, methylcellulose, talc, metallic stearates, silicone dioxide, plasticizers or the like. The admixture is used to coat a bead core such as a sugar sphere (or so-called "non-pareil") having a size of approximately 60 to 20 mesh.

An alternative procedure for preparing drug beads is by blending drug with one or more pharmaceutically acceptable excipients, such as microcrystalline cellulose, lactose, cellulose, polyvinyl pyrrolidone, talc, magnesium stearate, a disintegrant, etc., extruding the blend, spheronizing the extrudate, drying and optionally coating to form the immediate release beads.

### 4. Formulations for Mucosal and Pulmonary Administration

The probenecid, metabolites and analogs thereof, and pharmaceutical compositions thereof can be formulated for pulmonary or mucosal administration. The administration can include delivery of the composition to the lungs, nasal, oral (sublingual, buccal), vaginal, or rectal mucosa. In a particular embodiment, the composition is formulated for and delivered to the subject sublingually.

In some embodiments, the compound is formulated for pulmonary delivery, such as intranasal administration or oral inhalation. The respiratory tract is the structure involved in the exchange of gases between the atmosphere and the blood stream. The lungs are branching structures ultimately ending with the alveoli where the exchange of gases occurs. The alveolar surface area is the largest in the respiratory system and is where drug absorption occurs. The alveoli are covered by a thin epithelium without cilia or a mucus blanket and secrete surfactant phospholipids. The respiratory tract encompasses the upper airways, including the oropharynx and larynx, followed by the lower airways, which include the trachea followed by bifurcations into the bronchi and bronchioli. The upper and lower airways are called the conducting airways. The terminal bronchioli then divide into respiratory bronchiole, which then lead to the ultimate respiratory zone, the alveoli, or deep lung. The deep lung, or alveoli, is the primary target of inhaled therapeutic aerosols for systemic drug delivery.

Pulmonary administration of therapeutic compositions comprised of low molecular weight drugs has been observed, for example, beta-androgenic antagonists to treat asthma. Other therapeutic agents that are active in the lungs have been administered systemically and targeted via pulmonary absorption.

Nasal delivery is considered to be a promising technique for administration of therapeutics for the following reasons: the nose has a large surface area available for drug absorption due to the coverage of the epithelial surface by numerous microvilli, the subepithelial layer is highly vascularized, the venous blood from the nose passes directly into the systemic circulation and therefore avoids the loss of drug by first-pass metabolism in the liver, it offers lower doses, more rapid attainment of therapeutic blood levels, quicker onset of pharmacological activity, fewer side effects, high total blood flow per cm³, porous endothelial basement membrane, and it is easily accessible.

In some embodiments, the composition is formulated as an aerosol. The term aerosol as used herein refers to any preparation of a fine mist of particles, which can be in solution or a suspension, whether or not it is produced using a propellant. Aerosols can be produced using standard techniques, such as ultrasonication or high-pressure treatment.

Probenecid has been used to reduce nephrotoxicity of cidofovir when administered intravenuosly. Delivering cidofovir directly to the respiratory tract was also shown to be an effective prophylactic strategy that maximizes the tissue concentration at the site of initial viral replication, while minimizing its accumulation in the kidneys (Roy, et al., Antimicrob Agents Chemother., 47(9): 2933-2937 (2003)).

Carriers for pulmonary formulations can be divided into those for dry powder formulations and for administration as solutions. Aerosols for the delivery of therapeutic agents to the respiratory tract are known in the art. For administration via the upper respiratory tract, the formulation can be formulated into a solution, e.g., water or isotonic saline, buffered or un-buffered, or as a suspension, for intranasal administration as drops or as a spray. Preferably, such solutions or suspensions are isotonic relative to nasal secretions and of about the same pH, ranging e.g., from about pH 4.0 to about pH 7.4 or, from pH 6.0 to pH 7.0. Buffers should be physiologically compatible and include, simply by way of example, phosphate buffers. For example, a representative nasal decongestant is described as being buffered to a pH of about 6.2. One skilled in the art can readily determine a suitable saline content and pH for an innocuous aqueous solution for nasal and/or upper respiratory administration.

Preferably, the aqueous solution is water, physiologically acceptable aqueous solutions containing salts and/or buffers, such as phosphate buffered saline (PBS), or any other aqueous solution acceptable for administration to an animal or human. Such solutions are well known to a person skilled in the art and include, but are not limited to, distilled water, de-ionized water, pure or ultrapure water, saline, phosphate-buffered saline (PBS). Other suitable aqueous vehicles include, but are not limited to, Ringer's solution and isotonic sodium chloride. Aqueous suspensions may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

In another embodiment, solvents that are low toxicity organic (i.e. nonaqueous) class 3 residual solvents, such as ethanol, acetone, ethyl acetate, tetrahydrofuran, ethyl ether, and propanol may be used for the formulations. The solvent is selected based on its ability to readily aerosolize the formulation. The solvent should not detrimentally react with the compounds. An appropriate solvent should be used that dissolves the compounds or forms a suspension of the compounds. The solvent should be sufficiently volatile to enable formation of an aerosol of the solution or suspension. Additional solvents or aerosolizing agents, such as freons, can be added as desired to increase the volatility of the solution or suspension.

In one embodiment, compositions may contain minor amounts of polymers, surfactants, or other excipients well known to those of the art. In this context, "minor amounts" means no excipients are present that might affect or mediate uptake of the compounds in the lungs and that the excipients that are present are present in amount that do not adversely affect uptake of compounds in the lungs.

Dry lipid powders can be directly dispersed in ethanol because of their hydrophobic character. For lipids stored in organic solvents such as chloroform, the desired quantity of solution is placed in a vial, and the chloroform is evaporated under a stream of nitrogen to form a dry thin film on the surface of a glass vial. The film swells easily when reconstituted with ethanol. To fully disperse the lipid molecules in the organic solvent, the suspension is sonicated. Nonaqueous suspensions of lipids can also be prepared in absolute ethanol using a reusable PARI LC Jet+ nebulizer (PARI Respiratory Equipment, Monterey, CA).

Dry powder formulations ("DPFs") with large particle size have improved flowability characteristics, such as less aggregation, easier aerosolization, and potentially less phagocytosis. Dry powder aerosols for inhalation therapy are generally produced with mean diameters primarily in the range of less than 5 microns, although a preferred range is between one and ten microns in aerodynamic diameter. Large "carrier" particles (containing no drug) have been co-delivered with therapeutic aerosols to aid in achieving efficient aerosolization among other possible benefits.

Polymeric particles may be prepared using single and double emulsion solvent evaporation, spray drying, solvent extraction, solvent evaporation, phase separation, simple and complex coacervation, interfacial polymerization, and other methods well known to those of ordinary skill in the art. Particles may be made using methods for making microspheres or microcapsules known in the art. The preferred methods of manufacture are by spray drying and freeze drying, which entails using a solution containing the surfactant, spraying to form droplets of the desired size, and removing the solvent.

The particles may be fabricated with the appropriate material, surface roughness, diameter, and tap density for localized delivery to selected regions of the respiratory tract such as the deep lung or upper airways. For example, higher density or larger particles may be used for upper airway delivery. Similarly, a mixture of different sized particles, provided with the same or different active agents may be administered to target different regions of the lung in one administration.

Compositions and methods of preparing inhalation-type pharmaceutical compositions including probenecid are described in U.S. Published Application No. 2015/0272870.

Thus, formulations and methods of administering the disclosed compositions to the nasal mucosa and/or the lungs by intranasal delivery, and to the lung by oral inhalation are provided. With respect to intranasal delivery, the formulation and delivery device can be selected and prepared to drive absorption through the nasal mucosa or the lungs. The nasal mucosa is - compared to other mucous membranes - easily accessible and provides a practical entrance portal for small and large molecules (Bitter, et al., "Nasal Drug Delivery in Humans," in Surber, et al., (eds): Topical Applications and the Mucosa. Curr Probl Dermatol. Basel, Karger, 2011, vol 40, pp 20-35, Pires, et al., J Pharm Pharmaceut Sci., 12(3) 288 - 311, 2009, and Djupesland, Drug Deliv. and Transl. Res., 3:42-62 (2013) DOI 10.1007/s13346-012-0108-9). Intranasal administration offers a rapid onset of therapeutic effects, reduced first- pass effect, reduced gastrointestinal degradation and lung toxicity, noninvasiveness, essentially painless application, and easy and ready use by patients - particularly suited for children - or by physicians in emergency settings. Flu Mist^{®}, for example, is an exemplary effective nasal influenza vaccine spray.

Numerous delivery devices are available for intranasal administration. Devices vary in accuracy of delivery, dose reproducibility, cost and ease of use. Metered- dose systems provide dose accuracy and reproducibility. Differences also exist in force of delivery, spray patterns and emitted droplet size. The latter being important for drug deposition within the nasal cavity. Parameters can be can be modulated to enhance deposition while limiting the fraction of small particles able to bypass the nose and enter the lungs, or reduce deposition while increasing the fraction of small particles able to bypass the nose and enter the lungs.

The following aspects of nasal anatomy can influence drug delivery. During exhalation the soft palate closes automatically, separating the nasal and oral cavities. Thus, it becomes possible to use smaller particles in a nasal spray and still avoid lung deposition. Additionally, during closure of the soft palate there is a communication pathway between the two nostrils, located behind the walls separating the two passages. Under these circumstances, it is possible for airflow to enter via one nostril and leave by the other. This bidirectional delivery concept combines the two anatomical facts into one fully functional device. The device is inserted into one nostril by a sealing nozzle, and the patient blows into the mouthpiece. The combination of closed soft palate and sealed nozzle creates an airflow which enters one nostril, turns 180° through the communication pathway and exits through the other nostril (bidirectional flow). Since delivery occurs during exhalation, small particles cannot enter the lungs.

Particle size, flow rate and direction can be tuned for efficient delivery to the nasal mucosa. By adding an exit resistor to give additional control of the input pressure, it is possible to improve distribution to the sinuses and the middle ear. Manipulation of the flow pattern enables delivery to the olfactory region, thereby possibly achieving direct 'nose- to- brain' delivery. The 180- degree turn behind the septum will trap particles still airborne, allowing targeted delivery of cargo to the adenoid.

Strategies for enhancing drug absorption via nasal and pulmonary routes are also known in the art and can be utilized in the disclosed formulations and methods of delivery. Such strategies include, for example, use of absorption enhancers such as surfactants, cyclodextrins, protease inhibitors, and tight junction modulators, as well as application of carriers such as liposomes and nanoparticles. See, e.g., Ghadiri, et al., Pharmaceutics, 11(3): 113 (2019).

### 5. Formulations for Parenteral Administration

Probenecid, metabolites and analogs thereof, and pharmaceutical compositions thereof can be administered in an aqueous solution, by parenteral injection or infusion. The formulation may also be in the form of a suspension or emulsion. In general, pharmaceutical compositions are provided including effective amounts of the active agent(s) and optionally include pharmaceutically acceptable diluents, preservatives, solubilizers, emulsifiers, adjuvants and/or carriers. Such compositions include diluents sterile water, buffered saline of various buffer content (e.g., Tris-HCl, acetate, phosphate), pH and ionic strength; and optionally, additives such as detergents and solubilizing agents (e.g., TWEEN^{®} 20, TWEEN^{®} 80 also referred to as POLYSORBATE^{®} 20 or 80), anti-oxidants (e.g., ascorbic acid, sodium metabisulfite), and preservatives (e.g., Thimersol, benzyl alcohol) and bulking substances (e.g., lactose, mannitol). Examples of nonaqueous solvents or vehicles are propylene glycol, polyethylene glycol, vegetable oils, such as olive oil and corn oil, gelatin, and injectable organic esters such as ethyl oleate. The formulations may be lyophilized and redissolved/resuspended immediately before use. The formulation may be sterilized by, for example, filtration through a bacteria retaining filter, by incorporating sterilizing agents into the compositions, by irradiating the compositions, or by heating the compositions.

### III. Methods of Treatment

Any references in the description to methods of treatment refer to the compounds, pharmaceutical compositions and medicaments of the present invention for use in a method for treatment of the human (or animal) body by therapy (or for diagnosis). Methods of treating a viral infection in subject in need thereof are provided. In some embodiments, the virus can be one that causes a respiratory disease or illness. Thus, methods of treating a respiratory disease or illness, particularly in a subject infected with a virus are also provided.

The methods can include administering to a subject an effective amount of probenecid, a metabolite or analog thereof, or a pharmaceutically acceptable salt thereof to reduce viral replication, infection, or a combination thereof. In some embodiments, the amount is effective to reduce virus titer in the subject, reduce the host cell from assembling virus, reduce and/or limit hyper-inflammation associated with infection, and/or one or more severe respiratory symptoms.

In some embodiments, the subject has been, or will be, exposed to the virus. In some embodiments, the subject has been exposed to the virus or is experiencing an active viral infection.

In some embodiments, a viral infection is detected by PCR test designed to detect viral RNA in a sample from the subject, for example, a nasal swab, throat swab, saliva, or other bodily fluid; or a serological or immunodiagnostic test designed to detect antibodies, typically in a blood sample from the subject, that the body's immune system has produced in response to the infection.

The compositions can also be administered prophylactically to, for example, reduce or prevent the effects of future exposure to virus and the infection that may associated therewith. Thus, in some embodiments, the subject has not been exposed to the virus and/or is not yet experiencing an active viral infection. In some embodiments, the subject is a healthy subject.

In some embodiments, the subject has been in close contact with a subject that has tested positive for the virus. Such a subject may or may not be exhibiting one or more symptoms of an infection. Close contact may be or include, for example, being within 2 meters of someone who is infected for a total of 15 minutes or more, providing care at home to someone who is infected, having direct physical contact with the person (hugged or kissed them) who is infected, sharing eating or drinking utensils with an infected person, having been sneezed on, coughed on, or otherwise by contacted by respiratory droplets from an infected person.

In some embodiments, the subject was identified in contact-tracing as having been exposed to the virus, or one or more subjects infected therewith.

In some embodiments, the subject will be exposed to the virus. An exemplary subject is a healthcare worker that will treat infected people.

In some embodiments, treatment begins 1, 2, 3, 4, 5, or more hours, days, or weeks prior to or after exposure to the virus.

In some embodiments, the probenecid, a metabolite or analog thereof, or a pharmaceutically acceptable salt thereof is administered in an effective amount to reduce or prevent one or more symptoms of a viral infection. Symptoms include those of an acute respiratory illness, for example, fever, congestion in the nasal sinuses and/or lungs, runny or stuffy nose, cough, sneezing, sore throat, body aches, fatigue, shortness of breath, chest tightness, and wheezing when exhaling. Exemplary viruses and particular symptoms associated with infection thereby are discussed in more detail below. Most typically, the virus is a coronavirus.

In some embodiments, the subject does not have gout, need prolonged penicillin (or other antibiotic) serum levels, pelvic inflammatory disease, or gonorrhea.

In some embodiments, the subject has an influenza infection. See, e.g., Perwitasari, et al., Antimicrob Agents Chemother, 57(1):475-83 (2013). doi: 10.1128/AAC.01532-12.)). For example, in some embodiments, the subject has an influenza (e.g., influenza A, influenza B, influenza C, and/or influenza D) infection and an infection from another virus, such as a coronavirus. In some embodiments, the subject does not have an influenza viral infection.

Subjects can be male and/or female, adults (e.g., 18 or over), and/or children under 18.

### A. Exemplary Dosages and Regimens

Probenecid, metabolites and analogs thereof and pharmaceutically acceptable salts thereof can be administered to a subject in a pharmaceutical composition, such as those discussed above, and can be administered by parenteral (intramuscular, intraperitoneal, intravenous (IV) or subcutaneous injection), enteral, transdermal (either passively or using iontophoresis or electroporation), or transmucosal (nasal, pulmonary, vaginal, rectal, or sublingual) routes, as discussed in more detail above.

The precise dosage will vary according to a variety of factors such as subject-dependent variables (e.g., age, immune system health, clinical symptoms, route of delivery, etc.).

For treating gout, probenecid has been administered at 250 mg Per os/oral (PO) twice daily for 1 week; increasing to 500 mg PO twice daily to 2 g/day maximum with dosage increases of 500 mg.

For prolonging penicillin serum levels, probenecid has been administered at 500 mg PO four times daily.

For pelvic inflammatory disease probenecid has been administered at 1 g PO with 2 g cefoxitin intramuscular (IM) as single dose.

For gonorrhea, probenecid has been administered at 1 g PO with 2 g cefoxitin IM as single dose.

A typically pediatric (e.g., age: 2 to 14 years and weight less than 50 kg) administration as an adjuvant to antibiotic therapy is Initial: 25 mg/kg (or 0.7 g/m2) orally once; Maintenance: 40 mg/kg (or 1.2 g/m2) per day orally administered in 4 equally divided doses 4 times a day.

Thus, in general, by way of example only, dosage forms useful in the disclosed methods may include doses in the range of 0.1 mg to 3,000 mg; 25 mg to 2,000 mg; 25 mg to 1,000 mg; 50 mg to 1,000 mg; 100 mg to 1,000 mg; or 250 mg to 1,000 mg, with doses of 10 mg, 25 mg, 45 mg, 50 mg, 100 mg, 150 mg, 200 mg, 250 mg, 300 mg, 350 mg, 400 mg, 450 mg, 500 mg, 550 mg, 600 mg, 750 mg, and 1,000 mg being exemplary doses, which can be administered, for example, 1, 2, 3, 4, or 5 times daily, weekly, bi-weekly, etc., for 1, 2, 3, 4, or more weeks, and for example until symptoms improve or disappear. In some embodiments, a single treatment can be repeated 1, 2, 3, 4, 5, 6, 7, or more days, weeks, or months apart.

In some embodiments, the treatment regimen is similar to those describe above for, e.g., gout, prolonging penicillin serum levels, pelvic inflammatory disease, gonorrhea, etc.

In a particular embodiments, the probenecid or a metabolite or analog thereof or pharmaceutically acceptable salt thereof is administered as 250 mg twice per day.

The results below show that 2 mg/kg and 200 mg/kg dosages were both effective at treating hamsters *in vivo.* Thus, in some embodiments, the dosage is between 2 mg/kg and 200 mg/kg, inclusive.

As introduced above, recitation of ranges of values herein including the dosage ranges above and elsewhere herein, are merely intended to serve as a shorthand method of referring individually to each separate value falling within the range and each separate value is incorporated into the specification as if it were individually recited herein.

Dosing regimens may be, for example, intermittent dosing or continuous (e.g., constant infusion). The dosing regimens can include administrations of the same or different doses. Thus, the dosing regimen can include dose escalation, dose reduction, or a combination thereof.

In some embodiments, the composition is administered in a pulsed dosage regimen. Pulse dosing refers to dosing approach that produces escalating drug levels early in the dosing interval followed by a prolonged dose-free period. For example, in some embodiments, drug administration is frontloaded by means of, for example, 1, 2, 3, 4, or 5 sequential bolus administrations, after which drug levels are allowed to diminish until the next dose. In some embodiments, the serum drug level is allowed to diminish to about 0.

This type of drug delivery technology could offer therapeutic advantages such as reduced dose frequency and greater patient compliance. In comparison to intermittent dosing, pulse dosing front loads the drug, allowing an extended dose-free period during which drug concentration falls close to zero. However, unlike a single, large bolus dose (e.g., given once daily), short bursts of drug are separated by short dose-free periods, allowing the serum concentration to fluctuate (Ibrahim, et al., Antimicrobial Agents and Chemotherapy, 48(11):4195-4199 (2004)). In particular embodiments, pulse dosing is carried out by oral administration or intravenous administration. For example, in some embodiments, the therapy includes discontinuous/intermittent intravenous infusion of very high doses of probenecid, a metabolite or analog thereof, or a pharmaceutically acceptable salt thereof over a short period.

In some embodiments, a large bolus dose of probenecid, a metabolite or analog thereof, or pharmaceutically acceptable salt thereof is between about 1,000 mg and 5,000 mg inclusive, or any subrange or specific dosage there between.

The maximum recommended dosage for probenecid is 2 grams/day PO for adults, adolescents, and children of more than 50 kg, and 40 mg/kg/day (1.2 grams/m2/day) PO (not to exceed 2 grams/day PO) for adolescents and children of 50 kg or less. Thus, in some embodiments, administration does not exceed 5 g, 4 g, 3 g, or 2 g per day. In some embodiments, administration does not exceed 40 mg/kg/day. See also "probenecid - Drug Summary", the Prescribers' Digital Reference.

In some embodiments, a tablet for oral administration contains e.g., 500 mg of probenecid and optionally, one or more of the following inactive ingredients: microcrystalline cellulose, sodium lauryl sulfate, sodium starch glycolate, starch (corn), povidone, colloidal silicon dioxide, magnesium stearate, polyvinyl alcohol, titanium dioxide, polyethylene glycol, talc, D&C Yellow #10 Aluminum Lake, FD&C Yellow #6 Aluminum Lake, and FD&C Blue #2 Aluminum Lake.

In some embodiments, a subject is administered 500 mg of probenecid or a metabolite or analog thereof, or pharmaceutically acceptable salt thereof, orally (P.O.) twice daily (B.I.D.) for 14 days.

In some embodiments, treatment ends 0, 1, 2, 3, 4, or 5 days after the subject's symptom(s) resolve, the subject registers one, two, or more negative tests for viral infections (e.g., negative SARS-CoV-2/COVID-19 tests), or a combination thereof.

### B. Combination Therapies

In some embodiments, probenecid, a metabolite or analog thereof, or a pharmaceutically acceptable salt thereof is administered in combination with one or more additional active agents. The combination therapies can include administration of the active agents together in the same admixture, or in separate admixtures. Therefore, in some embodiments, the pharmaceutical composition includes two, three, or more active agents. Such formulations typically include an effective amount of probenecid, a metabolite or analog thereof, or a pharmaceutically acceptable salt thereof. In some embodiments, the second active agent is an antiviral (i.e., a second antiviral), a fever reducer, an anti-inflammatory, an analgesic, or a combination thereof. In a particular embodiment, the antiviral is oseltamivir phosphate (TAMIFLU^{®}). Tamiflu is a prescription medicine used to treat the flu (influenza) in people 2 weeks of age and older who have had flu symptoms. Probenecid or metabolites or analogs or pharmaceutical salts thereof may enhance the efficacy of antivirals like oseltamivir phosphate as it helps retain excretion of the drug during treatment.

Some products that may interact with this drug include: cancer chemotherapy, baricitinib, dyphylline, ketorolac, methotrexate, pyrazinamide, salicylates (e.g., high-dose aspirin), zidovudine, certain drugs removed from the body by the kidneys (such as ceftazidime/avibactam, dapsone, heparin, fosfomycin). Thus, in some embodiments, the subject is not administered one or more of these drugs while being treated with probenecid or metabolites or analogs or pharmaceutical salts thereof.

In some embodiments, one or more additional active agent is remdesivir.

### C. Exemplary Viruses and Symptoms

Exemplary viruses and symptoms of illness stemming from infection by the viruses that are treatable by the disclosed methods are also provided. The virus is SARS-CoV-2.

The current classification of coronaviruses recognizes 39 species in 27 subgenera, five genera and two subfamilies that belong to the family *Coronaviridae,* suborder *Cornidovirineae,* order *Nidovirales* and realm *Riboviria* (Coronaviridae Study Group of the International Committee on Taxonomy of Viruses, Nat Microbiol 2020. DOI: 10.1038/s41564-020-0695-z). They are enveloped viruses with a positive-sense single-stranded RNA genome and a nucleocapsid of helical symmetry. The genome size of coronaviruses ranges from approximately 26 to 32 kilobases, one of the largest among RNA viruses.

Coronaviruses cause diseases in mammals and birds. Most typically, alphacoronaviruses and betacoronaviruses infect mammals, while gammacoronaviruses and deltacoronaviruses primarily infect birds. At least seven of these viruses can infect people: 229E (alpha) NL63 (alpha) OC43 (beta) HKU1 (beta) MERS-CoV (beta) virus, SARS-CoV (beta), and SARS-CoV-2 (beta).

In preferred embodiments, the subject is a human. In humans, coronaviruses can cause respiratory tract infections that can range from mild to lethal. Mild illnesses include some cases of the common cold, while more lethal varieties can cause SARS, MERS, and COVID-19 (i.e., caused by SARS-CoV-2).

The subject may have one or more symptoms characteristic of SARS, or COVID-19.

SARS (i.e., SARS-CoV) usually begins with flu-like signs and symptoms such as fever, chills, muscle aches, headache and occasionally diarrhea. After about a week, signs and symptoms include fever of 100.5 F (38 C) or higher, dry cough, and shortness of breath.

Reported illnesses from COVID-19 (i.e., caused by SARS-CoV-2) have ranged from mild symptoms to severe illness and death for confirmed cases. The most common symptoms are fever, tiredness, dry cough, anosmia (loss of taste and/or smell) and shortness of breath. Runny nose, vomiting, diarrhea, skin rash (particularly on toes and fingers), sore throat, fatigue, muscle or body aches, headache, and sore throat have also been reported. These symptoms may appear 2-14 days after exposure.

In some embodiments, the subject has an underlying condition such as asthma, heart disease, diabetes, cancer, chronic lung disease, chronic heart disease, chronic kidney disease, or a combination thereof.

The age of the subject can range from the young childern, including pediatric subjects, to the elderly. In some embodiments, the subject is at least 2 years old, particularly if having one or more symptoms, e.g., cough fever, and/or other discussed herein, etc. Treatment may be particularly indicated if the subject is male and/or over the age of 40, 50, 60, 70 or 80. In some embodiments, the subject is obese (BMI of over 30, where BMI is Body Mass Index, calculated as a person's weight in kilograms divided by the square of his or her height in meters).

In some embodiments, the subject has at least one mild or moderate COVID-19 symptom such as fever or chills, cough, shortness of breath or difficulty breathing, fatigue, muscle or body aches, headache, new loss of taste or smell, sore throat, congestion or runny nose, nausea, vomiting or diarrhea for 6, 5, 4, 3, 2, or 1 day(s) or less prior has treatment.

In other embodiments, the subject is a non-human mammal or a bird.

The virus is The sequence WIV04/2019, belonging to the GISAID S clade / PANGO A lineage / Nextstrain 19B clade, is thought to most closely reflect the sequence of the original SARS-CoV-2 infecting humans. It is known as "sequence zero", and is widely used as a reference sequence. Subsequent to the initial isolate from Wuhan, China, numerous SARS-CoV-2 virus sequence variants of WIV04/2019 have been identified, some of which may be of particular importance due to their potential for increased transmissibility, increased virulence, and reduced effectiveness of vaccines against them. SARS-CoV-2 having a variation of the WIV04/2019 sequence include, but are not limited to:
**B.1.1.7 lineage** (a.k.a. 20I/501Y.V1 Variant of Concern (VOC) 202012/01). This variant has a mutation in the receptor binding domain (RBD) of the spike protein at position 501, where the amino acid asparagine (N) has been replaced with tyrosine (Y). The shorthand for this mutation is N501Y. This variant also has several other mutations, including: 69/70 deletion: occurred spontaneously many times and likely leads to a conformational change in the spike protein.
P681H: near the S1/S2 furin cleavage site, a site with high variability in coronaviruses. This mutation has also emerged spontaneously multiple times.

**B.1.351 lineage** (a.k.a. 20H/501Y.V2). This variant has multiple mutations in the spike protein, including K417N, E484K, N501Y. Unlike the B.1.1.7 lineage detected in the UK, this variant does not contain the deletion at 69/70.

**P.1 lineage** (a.k.a. 20J/501Y.V3). The P.1 variant is a branch off the B.1.1.28 lineage that was first reported by the National Institute of Infectious Diseases (NIID) in Japan in four travelers from Brazil, sampled during routine screening at Haneda airport outside Tokyo. The P.1 lineage contains three mutations in the spike protein receptor binding domain: K417T, E484K, and N501Y.

Other lineages and mutations of interest include, but are not limited to, B.1.1.207, B.1.429, B.1.427, B.1.525, and other two-mutation (e.g., N501T-G142D), or three-mutation (e.g., N501T-G142D-F486L) variants in the Spike protein. All of these lineages and sequence alternatives relative to the WIV04/2019 strain are also considered SARS-CoV-2 virus. In some embodiments, SARS-CoV-2 is a strain or isolate with with elevated or potentially elevated risk for causing human disease relative to WIV04/2019. See, e.g., Science Brief, Emerging SARS-CoV-2 variants (CDC website, Updated Jan. 28, 2021), Horby, et al., "NERVTAG note on B.1.1.7 severity." SAGE meeting report. January 21, 2021; Wu, et al. "mRNA-1273 vaccine induces neutralizing antibodies against spike mutants from global SARS-CoV-2 variants." bioRxiv. Posted January 25, 2021; Xie, et al., "Neutralization of N501Y mutant SARS-CoV-2 by BNT162b2 vaccine-elicited sera." bioRxiv. Posted January 7, 2021; Greaney, et al. "Comprehensive mapping of mutations to the SARS-CoV-2 receptor-binding domain that affect recognition by polyclonal human serum antibodies." bioRxiv. [Preprint posted online January 4, 2021]; Weisblum, et al., "Escape from neutralizing antibodies by SARS-CoV-2 spike protein variants." eLife 2020;9:e61312; Resende, at al. "Spike E484K mutation in the first SARS-CoV-2 reinfection case confirmed in Brazil," 2020. [Posted on virological.org on January 10, 2021]

Various strains and isolates of the foregoing viruses are known and include the representative genomic sequences provided as, for example, SEQ ID NOS:1-4, the accession numbers provided herein, and those sequences and accession numbers provided in, e.g., Coronaviridae Study Group of the International Committee on Taxonomy of Viruses, Nat Microbiol 2020. DOI: 10.1038/s41564-020-0695-z), as well as NCBI and GISAID which provide hundreds of SARS-CoV-2 sequences.

In some embodiments, the SARS-CoV-2 has 1, 2, 3, 4, 5, 6, 7, 8, 9, 10, 11, 12, 13, 14, 15, 16, 17, 18, 19, 20, 21, 22, 23, 24, 25, or more mutations in its spike protein relative to e.g., WIV04/2019, SEQ ID NO:1, and/or SEQ ID NO:2 or another reference sequence such as those provided therein. The spike protein sequence for SEQ ID NO:1 is 




 
















 (SEQ ID NO:5)
 which is encoded by 21563..25384 of GenBank: MN908947.3 (SEQ ID NO:1), /gene="S", /note="structural protein", /codon_start=1 /product="surface glycoprotein", /protein_id="QHD43416.1").

In some embodiments, the spike protein of the SARS-CoV-2 has at least 70%, 75%, or 80%, preferably at least 85%, more preferably at least 86%, 87%, 88%, 89%, 90%, 91%, 92%, 93%, 94%, 95%, 96%, 97%, 98%, 99%, or 100% sequence identity to the spike protein of one or more of SEQ ID NOS:1 or 2, or another viral accession number provided herein.

In some embodiments, the spike protein of the SARS-CoV-2 has at least 70%, 75%, 80%, 85%, 86%, 87%, 88%, 89%, 90%, 91%, 92%, 93%, 94%, 95%, 96%, 97%, 98%, 99%, or 100% sequence identity to SEQ ID NO:5.

Mutations can be substitutions, insertions, deletions, or a combination thereof. Exemplary mutations include thoses discussed herein, e.g., one or more of the following mutations in the spike protein sequence:
H69, optionally deletion thereof;
V70, optionally deletion thereof;
G142, optionally G142D;
K417, optionally K417N or K417T;
E484, optionally E484K;
F486, optionally F486L; and/or
N501 mutation, optionally N501Y or N501T.
These mutations are provided individually and in all combinations. These residues are illustrated with with bold/italics/shading in SEQ ID NO:5 above.

In some embodiments, the SARS-CoV-2 is of the B.1.1.7, B.1.351, P.1, B.1.1.207, B.1.429, B.1.427, or B.1.525 lineage.

In an exemplary embodiment, the SARS-CoV-2 is isolate USA/CA_CDC_5574/2020, or another isolate sharing one or mutations therewith relative to the originoal Wuhan isolate. Under the nomenclature system introduced by GISAID (Global Initiative on Sharing All Influenza Data), SARS-CoV-2, isolate USA/CA_CDC_5574/2020 is assigned lineage B.1.1.7 and GISAID clade GR using Phylogenetic Assignment of Named Global Outbreak LINeages (PANGOLIN) tool (GISAID website, 3. Rambaut, et al., Nat. Microbiol. 5 (2020): 1403-1407. PubMed: 32669681; Mercatelli, et al., Front. Microbiol. (2020): doi.org/10.3389/fmicb.2020.01800. PubMed: 32793182. The complete genome of SARS-CoV-2, isolate USA/CA_CDC_5574/2020 has been sequenced (GISAID: EPI_ISL_751801). The following mutations are present in the clinical isolate:
Spike A570D, Spike D614G, Spike D1118H, Spike H69del, Spike N501Y, Spike P681H, Spike S982A, Spike T716I, Spike V70del, Spike Y145del, M (Membrane protein) V70L, N (Nucleocapsid protein) D3L, N G204R, N R203K, N S235F, NS3 T223I, NS8 (Non-structural protein 8) Q27stop, NS8 R52I, NS8 Y73C, NSP3 (Non-structural protein 3) A890D, NSP3 I1412T, NSP3 T183I, NSP6 (Non-structural protein 6) F108del, NSP6 G107del, NSP6 S106del, NSP12 (Non-structural protein 12) P323L, NSP13 (Non-structural protein 13) A454V, NSP13 K460R. One additional SNP in ORF1ab L3826F was reported in the deposited passage two virus, in comparison to the clinical specimen. See also BEI Resources, Catalog No. NR-54011.

The disclosed compositions and methods might also be used to treat other strains of coronavirus, particularly SARS and MERS coronaviruses. In some embodiments, the (DNA sequence) of the viral genome has a sequence at least 70%, 75%, or 80%, preferably at least 85%, more preferably at least 86%, 87%, 88%, 89%, 90%, 91%, 92%, 93%, 94%, 95%, 96%, 97%, 98%, 99%, or 100% sequence identity to one or more of SEQ ID NOS:1, 2, 3, or 4, or another viral accession number provided herein, or a sequence or accession number provided in Coronaviridae Study Group of the International Committee on Taxonomy of Viruses, Nat Microbiol 2020. DOI: 10.1038/s41564-020-0695-z. It will be appreciated that the sequences are provided as DNA sequences, but the viral genome itself will typically have the corresponding RNA sequences. Thus, the corresponding RNA sequences are also expressly provided herein.

GenBank Accession No. MN908947.3, NCBI Accession No. NC_045512.2. provides the (DNA) genomic sequence for SARS-CoV-2 (Severe acute respiratory syndrome coronavirus 2 isolate Wuhan-Hu-1, complete genome): (SEQ ID NO:1).

GenBank Accession No. MN985325.1 provides the (DNA) genomic sequence for SARS-CoV-2 (Severe acute respiratory syndrome coronavirus 2 isolate 2019-nCoV/USA-WA1/2020, complete genome): (SEQ ID NO:2).

GenBank Accession No. GenBank: AY274119.3 provides the (DNA) genomic sequence for SARS-CoV (Severe acute respiratory syndrome-related coronavirus isolate Tor2, complete genome): (SEQ ID NO:3).

GenBank Accession No. GenBank: JX869059.2 provides the (DNA) genomic sequence for MERS-CoV (Human betacoronavirus 2c EMC/2012, complete genome): (SEQ ID NO:4).

In some embodiments, the subject is diagnosed with a positive SARS-CoV-2 viral test result and has at least one mild or moderate COVID-19 symptom (i.e. fever or chills, cough, shortness of breath or difficulty breathing, fatigue, muscle or body aches, headache, new loss of taste or smell, sore throat, congestion or runny nose, nausea, vomiting or diarrhea) for 6 days or less prior to the first administration of the probenecid, metabolite or analog thereof or pharmaceutically acceptable salt thereof.

### Examples

### Example 1: Probenecid Reduces SARS-CoV-2 Plaque Formation in vitro

### Materials and Methods

### Plaque Reduction Assay

### Prophylactic treatment

Vero E6 cells (ATCC CRL-1586) were plated in 12-well plates at 5x10³cells/well and incubated overnight.

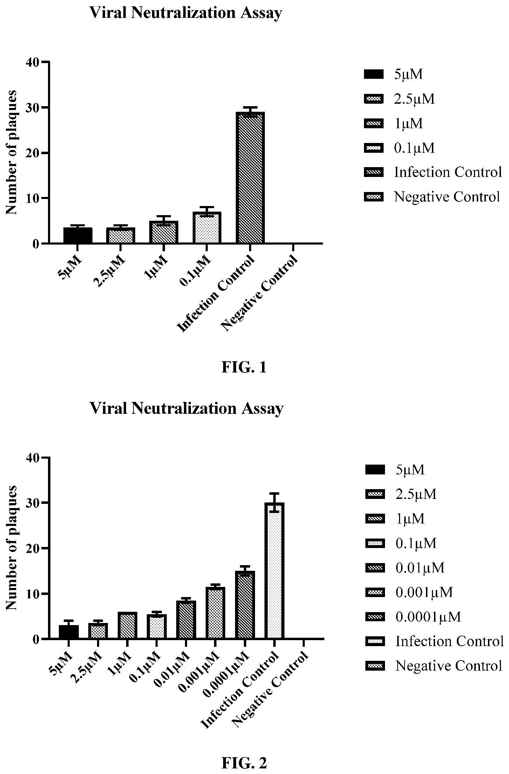

Cells were washed 1x with PBS and probenecid at 0.1 µM, 1 µM, 2.5 µM, or 5 µM, or vehicle control was added to the wells in culture media and incubated for 24 hours (Figure 1). All wells were normalized to 0.05% DMSO. Each concentration was completed in duplicate.

Following pre-treatment, media was discarded and cells were replenished with media containing probenecid (as above) and SARS-CoV-2 (stock grown from Isolate USA-WA1/2020, BEI Resources Catalogue Ref. Number NR-52281). The complete genome of SARS-CoV-2, USA-WA1/2020 has been sequenced (the isolate - GenBank Accession Number: MN985325 and after one passage in Vero cells - GenBank Accession Number: MT020880 and after four passages in Vero cells - GenBank Accession Number: MT246667).

Cells were infected at a MOI of 0.01 for 4 days. Post-infection cells were fixed and stained to visualize plaques. Plaques were quantified manually (Figure 1).

Statistical analysis was performed using one-way ANOVA with Dunnett's multiple comparisons test (treatment vs DMSO). P<0.05* using a repeated measures design.

In another experiment, probenecid at 0.0001 µM, 0.001 µM, 0.01 µM, 0.1 µM, 1 µM, 2.5 µM, or 5 µM (Figure 2) was added. Vero E6 cells were plated in a 6 well plate at 8E5cells/well and incubated overnight. Cells were washed once with PBS and the compound was added to the wells in culture media and incubated for 24 hours. All wells were normalized to 0.05% DMSO. Each concentration was completed in duplicate. Following pre-treatment, media was discarded and cells were replenished with media containing drug (as above) and SARS-CoV-2. Cells were infected at a MOI of 0.01 for 4 days. Post-infection the cells were fixed and stained to visualize plaques. Plaques were quantified (Figure 2).

### Therapeutic treatment

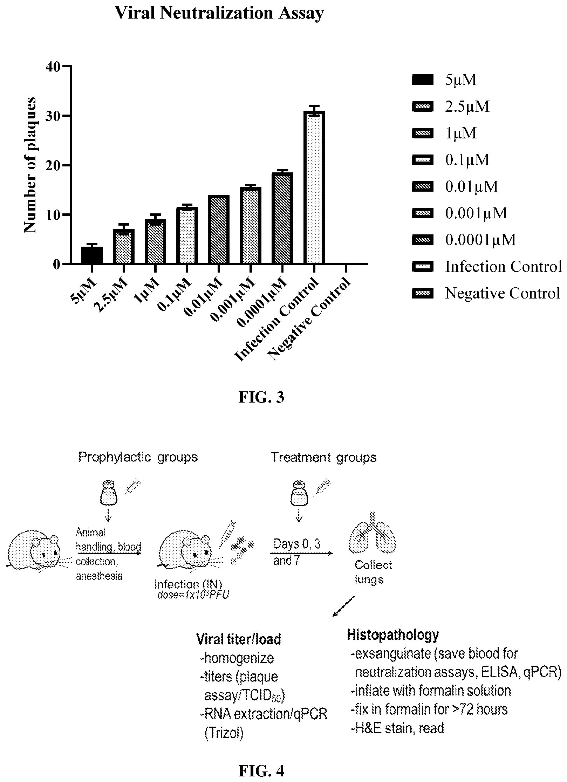

Vero E6 cells were plated in a 6 well plate at 8E5cells/well and incubated overnight. Cells were washed once with PBS and infected with virus at a MOI of 0.01 for 1 hour. Following 1 hour infection probenecid at 0.0001 µM, 0.001 µM, 0.01 µM, 0.1 µM, 1 µM, 2.5 µM, or 5 µM was added to the wells in overlay media and incubated 4 days. Post-infection the cells were fixed and stained to visualize plaques. Plaques were quantified (Figure 3).

### Results

The effect of probenecid on viral replication was investigated using an *in vitro* plaque formation assay.

Probenecid pre-treatment resulted in a dose dependent reduction in plaque formation for concentrations tested (5µM - 0.1µM) in two independent experiments. 5µM - 0.1µM reduced plaque formation from ~89% to 72% (Figure 1), respectively, compared to DMSO treated infected control.

In another experiment, probenecid pre-treatment resulted in a dose dependent reduction in plaque formation for concentrations tested (5µM-0.0001µM) in two independent experiments. 5µM - 0.0001µM reduced plaque formation from ~93% to 50%, respectively, (Figure 2), compared to DMSO treated infected control.

In another experiment, probenecid post-treatment resulted in a dose dependent reduction in plaque formation for concentrations tested (5µM-0.0001µM). 5µM - 0.0001µM reduced plaque ~90% to 40% (Figure 3), respectively, compared to DMSO treated infected control.

These results show that probenecid significantly reduces viral titer/plaque formation with 24 hour pretreatment at the concentrations tested. These results also show that probenecid significantly reduces viral titer/plaque formation with post-treatment at the concentrations tested.

### Example 2: Prophylactic and Therapeutic Probenecid Treatment Reduces Viral Load In Vivo.

### Materials and Methods

84 hamsters were obtained and used as subject for assays to determine if probenecid has prophylactic and/or therapeutic effects on SARS-CoV-2 (stock grown from Isolate USA-WA1/2020, BEI Resources Catalogue Ref. Number NR-52281) infection *in vivo.*

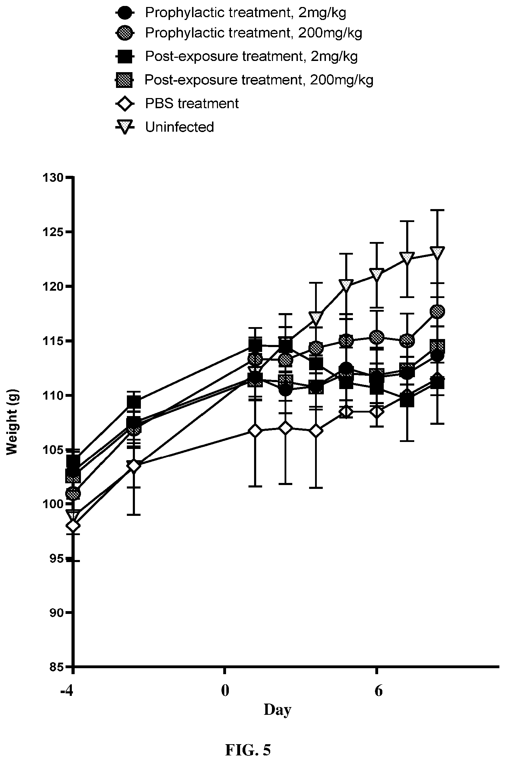

Prophylactic probenecid treatment was on Day -1. Infection (1x10³ PFU) was induced by delivering virus intranasally in a 50 µl volume on Day 0. Post-infection/therapeutic probenecid treatment was on Day 2. Animals were euthanized and tissue was collected for analysis on Day 0, Day 3, and Day 7. See Figure 5.

### Groups

- Uninfected (n=2/time point)
- PBS infected (n=2/time point)
- Prophylactic, 24-hr pre-infection, 200mg/kg (n=6/time point)
- Prophylactic, 24-hr pre-infection, 2mg/kg (n=6/time point)
- Treatment, 48-hr post-infection,
- 200mg/kg (n=6/time point)
- Treatment, 48-hr post-infection, 2mg/kg (n=6/time point)
- n=3 time points (days 0, 3, 7 post-infection)
- n=84 animals total

### Endpoints

- Weights and clinical symptoms (daily)
- Viral titer/load (n=3/group/time point)
   ∘ homogenize lungs
   ∘ titers (plaque assay and TCID₅₀)
   ∘ RNA extraction and qPCR
- Histopathology (n=3/group/time point)
   ∘ exsanguinate (save serum for neutralization assays, ELISA, qPCR)
   ∘ inflate with formalin solution
   ∘ fix in formalin for >72 hours
   ∘ H&E stain, read

### TCID₅₀ Assay

TCID50 was completed in 96-well plates of Vero E6 cells. The lung homogenate was serial diluted and 200ul was added to each well. The plates were incubated for 72 hours. The plates were then fixed and stained.

### Plaque Assay

Plaque assay was completed in 12 well plates of Vero E6 cells. The lung homogenate was serial diluted and 500ul was added to each well. The plates were incubated for 96 hours. The plates were then fixed and stained.

### Results

Hamsters were weighed prior to treatment and then every day following infection (Day 0). The uninfected group continually gained weight throughout the study. The other animals stopped gaining weight after infection but did not significantly lose weight. See Figure 5.

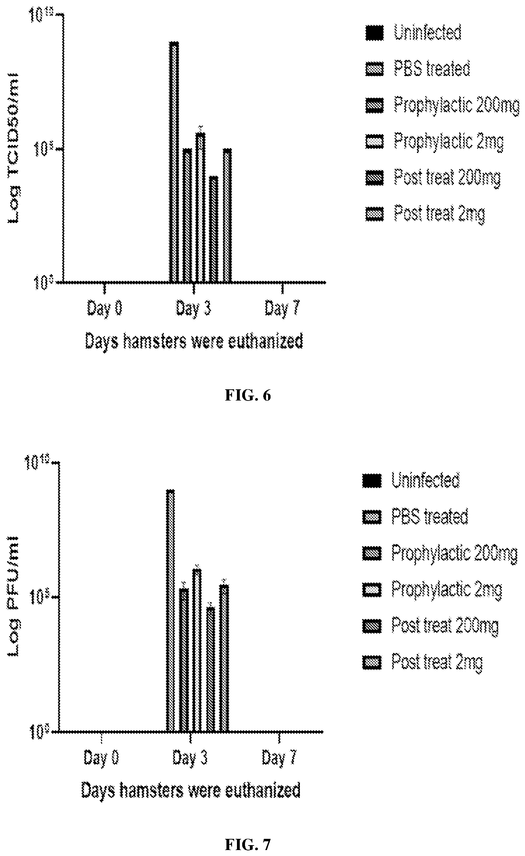

Results show that no virus is detected in the lungs of uninfected hamsters. At day 3 post-infection (pi) all probenecid-treated hamsters had low lung virus titers (1E5) compared to PBS-treated (1E9+). The results shows that probenecid decreases lung virus titers by several *logs* of virus. 2 mg and 200 mg treatment was similarly effective despite the hamsters receiving a single dose. Prophylactic and therapeutic treatment worked similarly. TCID₅₀ and plaque data were nearly identical. There was no detectable lung virus at day 7 post infection. See Figures 6 and 7.

### Example 3: Probenecid Improves Recovery from SARS-CoV-2 Infection in Human Subject.

### Materials and Methods

In an open label, ten patients, twenty-eight (28) day, Investigator Initiated Study (IIS) in non-hospitalized patients with mild to moderate SARS-CoV-2 infection were treated with probenecid ("clinical trial").

### Inclusion Criteria:

- Non-hospitalized patients diagnosed with a positive SARS-CoV-2 RNA test within 5 days prior to Screening/Baseline/First Dose Visit 1.
- Patient has presented within 5 days or less of screening with at least one mild or moderate COVID-19 symptom (i.e. fever or chills, cough, shortness of breath or difficulty breathing, fatigue, muscle or body aches, headache, new loss of taste or smell, sore throat, congestion or runny nose, nausea, vomiting or diarrhea).
- Men and women, ≥18 years of age.
- Patient has estimated glomerular filtration rate (eGFR) ≥30 mL/min using the Cockcroft-Gault formula.
- Willing to participate in this study, signed Informed Consent and willing to participate in regular follow-up during the study.
- Able to understand and cooperate with study procedures.

### Exclusion criteria:

- Patient is hospitalized or requires hospitalization at time of enrollment.
- Patient is immune compromised with human immunodeficiency virus (HIV) or other "immunocompromised" condition.
- Patient suffers from severe cognitive impairment or mental illness.
- Females of childbearing potential who are nursing, pregnant, intending to become pregnant or intending to nurse during the time of the study, or who have a positive urine pregnancy test at screening (a negative result is mandatory for eligibility).
- Patient who may be allergic to the study drug and the PI believes the study drug is not appropriate for the patient.
- Participating in other clinical studies at the same time.
- In the investigators' opinion, patients who have medical conditions that could interfere with drug metabolism or absorption (e.g., short bowel syndrome, Crohn's disease, etc.).
- Evidence of significant medical condition or laboratory finding which, in the opinion of the investigator, makes it undesirable for the patient to participate in the trial, such as malignant tumors, history of uric acid kidney stones, known blood dyscrasias or other unstable/clinically significant illness.
- Known history of chronic obstructive pulmonary disease (COPD).
- Received any investigational treatment with an anti-COVID-19 therapy in the past 30 days, for example, remdesivir, camostat, ritonavir, hydroxychloroquine, azithromycin, ruxolitinib or corticosteroids.
- Received organ, stem cell, or bone marrow transplantation.

### Analysis Variables:

- Screening variables consist of baseline patient characteristics: complete physical exam, height and weight, vital signs, hematology, chemistry panel and urine pregnancy test.
- Efficacy variables consist of qualitative SARS-CoV-2 test, SpO2, body temperature and respiratory symptom grading (WHO clinical status on ordinal scale), time to hospital admission, duration of hospitalization.
- Routine and special safety variables consist of assessment of AEs/SAEs, clinical laboratory assessments: CBC, CMP, TG, AA, OM-3 ratio, EPA and DHA, hs-CRP, LDH, CPK, ALT, creatinine, ferritin, fibrinogen, D-dimer and von Willebrand factor (vWF) antigen.

Patients positive for COVID-19 were screened and given a physical examination on Day 1 and retested using iAMP qualitative SARS-CoV-2 RNA test. Safety laboratory tests were also performed on Day 1 that included CBC with differential, Comprehensive Metabolic Profile (CMB) and inflammatory markers for COVID-19 (i.e., high sensitivity (hr-CRP), ferritin, d-dimer, CPK, and LDH).

On Day 2, after review of safety laboratory values, the primary investigator determined if patients could continue in the study and the first dose of study drug probenecid 500 mg was administered. Patients received 500 mg twice daily for 14 days, Study Day 2 to Study Day 15.

Patients returned to the clinic on Study Day 5, 10, 15, and completed on Study Day 28.

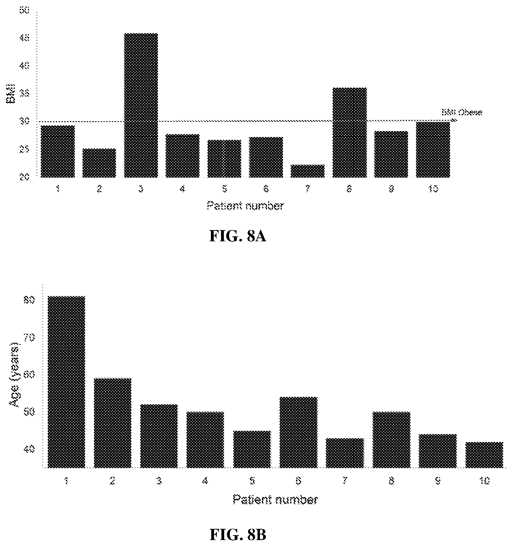

The age demographics in this study ranged from 42 to 81 years. Patient 1, 3 and 8 had existing comorbidities. Patients enrolled in the study had the characteristics in Table 1 and Figures 8A and 8B.

**Table 1: Patient Characteristics**

| Patient Characteristics | | | | | | | | | | |
|---|---|---|---|---|---|---|---|---|---|---|
| | 1 | 2 | 3 | 4 | 5 | 6 | 7 | 8 | 9 | 10 |
| Patient Initials | KHG | LAS | N-B | ICB | J-C | C-T | AGG | WTB | J-J | S-J |
| DOB (mm/dd/yy) | 10/26/39 | 8/21/61 | 5/20/68 | 10/18/70 | 11/8/75 | 9/28/66 | 11/16/77 | 12/12/70 | 5/30/76 | 2/6/78 |
| Age | 81 | 59 | 52 | 50 | 45 | 54 | 43 | 50 | 44 | 42 |
| Ethnicity | White | White | White | White | White | White | White | White | White | White |
| Sex | M | F | M | M | F | M | F | M | M | F |
| Height (inches) - visit 1 | 69 | 62 | 70 | 67 | 65 | 69 | 63 | 71 | 68 | 67 |
| Weight (pounds) - visit 1 | 190 | 136.2 | 302.4 | 171.8 | 155.4 | 177.2 | 123.7 | 245.6 | 180.8 | 185 |
| BMI | 29.3 | 25.2 | 45.9 | 27.7 | 26.7 | 27.3 | 22.3 | 36.1 | 28.3 | 29.9 |
| Comorbities | HTN | HCL | Obesity | | | | | CVD | | |
| | | | | | | | | HTN | | |
| | | | | | | | | HCL | | |

### Results

A clinical trial was designed to test the effect of probenecid on SARS-CoV-2 infection in human subjects. Data was monitored for the 10 patients treated through 14 days of treatment (Day 15) and included: positive/negative SARS-CoV-2 testing, symptom improvements, safety, coagulation, inflammatory lab values, and adverse events, each of which is discussed in more detail below.

### SARS-CoV-2 testing

In a recent in silico study, authors conducted a literature search to understand viral shedding of SARS-CoV-2. Seventy-seven (77) studies/reports were eligible for inclusion. The review found the pooled median duration of viral shedding from respiratory sources for mild to moderate illness was 17.2 days (Cevik, et al., "SARS-CoV-2, SARS-CoV, and MERS-CoV viral load dynamics, duration of viral shedding, and infectiousness: a systematic review and meta-analysis," The Lancet, 2 (1), E13-E22 (2021), DOI:10.1016/52666-5247(20)30172-5). In treated patients from this IIS study of probenecid, viral shedding was lower than the pooled median for all but one patient, Patient 003.

Of the 10 patients enrolled in the study having a positive SARS-CoV-2 test, 2 converted to negative on Day 4, four on Day 9, and nine on Day 14 of treatment.

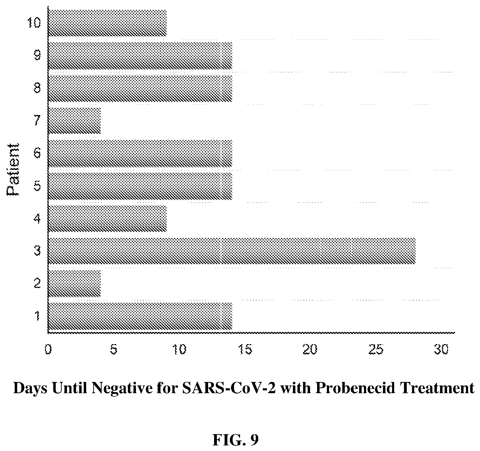

SARS-CoV-2 negative test results are summarized in Figure 9. An observation of weight as a comorbidity, Patient 003 was >300 pounds and remained positive at day 14 of treatment.

### Improvement in Symptoms

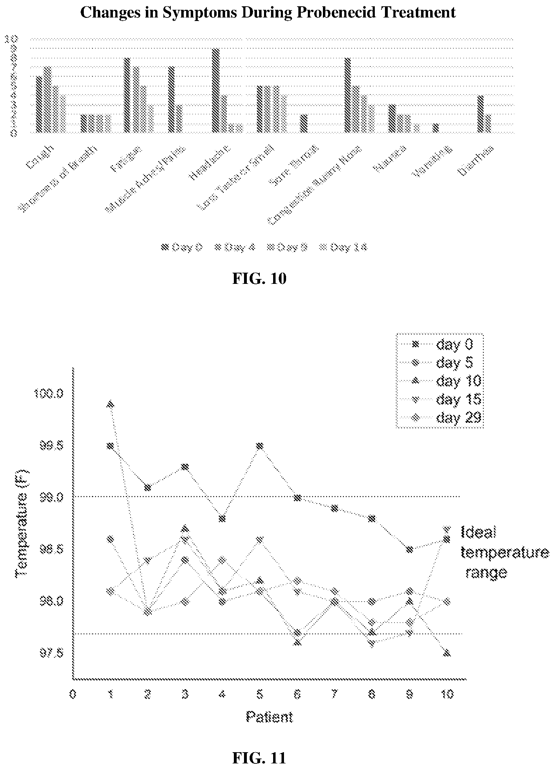

Improvement in symptoms is summarized in Figure 10. Two symptoms persisted: shortness of breath and loss of taste or smell. It is not uncommon with SARS-CoV-2 infection for these symptoms to persist over time.

Patient 001, an eighty-one (81) year old male, with a comorbidity of hypertension, was hospitalized for shortness of breath from day 5 to day 11 of treatment. The patient continued on the study drug, received 2-3 liters of oxygen during the stay, and returned home on 2 liters of oxygen via nasal canula. While hospitalized the patient received dexamethasone and Zithromax, but no convalescent plasma nor remdesivir.

### Indicators Outcome and Inflammatory Response

Low lymphocytes, high fibrinogen, and elevated d-dimer may be indicators of a worse outcome. Patient 001 had low lymphocytes values at enrollment through Study Day 15 at which time they returned to normal. Patient 001 also had elevated fibrinogen and d-dimer through Study Day 15 and elevated LDH at Day 15.

Patient 005 also went to the emergency room on study day 15. She was diagnosed with pneumonia but discharged to home because her oxygen saturation was normal. She was given steroids and an antibiotic. Patient 005 had elevated d-dimer through Study Day 15.

Figure 11 shows the resolution in body temperature across all patients.

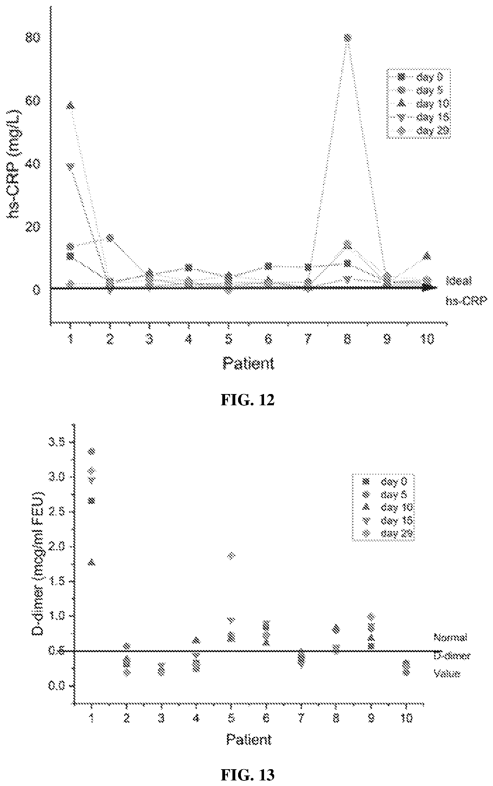

High sensitivity C-reactive protein (hs-CRP) is an indicator of early inflammatory response to SARS-CoV-2. A value of > 3.1 is indicative of infection and inflammation. The hs-CRP values spiked in 2 patients but decreased while on treatment. The hs-CRP did not return to normal by Study Day 15 (14^{th} day of treatment). Figure 12 shows all patients' hs-CRP values and the response to treatment.

D-dimer is another indicator of early coagulopathy and inflammatory response related to SARS-CoV-2 infection. The normal range for d-dimer is below 0.5 mcg/ml. All ten patients showed signs of SARS-CoV-2 related early coagulopathy and inflammation. Eight of 10 patients were below 1 mcg/ml during the treatment phase of the study. Patient 003 had the highest value at enrollment and approached normal during treatment. Patient 001 d-dimer values were high through Study Day 15. Figure 13 shows the d-dimer values for all patients during treatment.

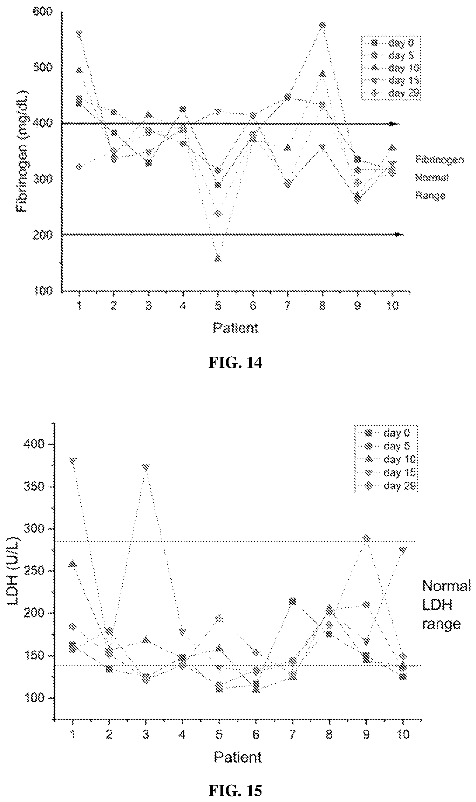

Elevated fibrinogen, greater than 425 mg/dL is an indication of the development of coagulation or blood clots. Three patients had fibrinogen values that exceeded the upper limit of normal. As mentioned above, Patient 001 had elevated fibrinogen levels through Study Day 15. Patient 007 had elevated fibrinogen that decreased to normal by Treatment Day 9 and Patient 008 by Treatment Day 14. Figure 14 shows all patients' fibrinogen values.

In this age population, elevated Lactate Dehydrogenase (LDH) greater than 250 U/L, is a sign of tissue damage. Patient 001who required hospitalization and oxygen had increased LDH after discharge. Patient 010 had a slightly elevated level on Treatment Day 14. LDH for all patients is shown in Figure 15.

### Conclusions

This open label study of ten consecutively enrolled non-hospitalized patients has shown treatment with probenecid decreased viral shedding of SARS-CoV-2 below the pooled median of 17.2 days (Cevik, et al., "SARS-CoV-2, SARS-CoV, and MERS-CoV viral load dynamics, duration of viral shedding, and infectiousness: a systematic review and meta-analysis," The Lancet, 2 (1), E13-E22 (2021), DOI:10.1016/S2666-5247(20)30172-5) Although a scored grading system was not used, patient symptoms showed improvement. Coagulation and inflammatory markers were mostly maintained within normal ranges with the exception of one patient (Patient 001), who upon enrollment had several elevated biomarkers.

### Example 4: Probenecid Compares Favorably with Remdesivir in an in vitro Plaque Assay.

Remdesivir is a nucleotide prodrug of an adenosine analog antiviral approved by the FDA for the treatment of COVID-19 requiring hospitalization on October 22, 2020. Experiments were designed to compare the antiviral activity of probenecid, remdesivir, and a combination of probenecid and remdesivir. Prophylactic and therapeutic (at the time of the infection) experiments were carried out generally as discussed above in Example 1, in NHBE and Vero cells. All experiment were performed >3 independent times in triplicate. The results are presented in Figures 16A-20F.

Results showed showed a dose-dependent decrease in virus plaque formation when treated prophylactically or therapeutically with probenicid compared to controls.

Prophylactic treatment with probenecid prevents SARS-CoV-2 replication in mammalian cells (e.g., similar to results presented in Example 1, Vero E6 cells treated with different concentrations of probenecid inhibited SARS-CoV-2 replication).

Under prophylactic and therapeutic conditions, and in both cell types tested, probenecid has lower IC50s and IC90s than remdesivir.

Results also show that co-administration of 0.1 µM probenecid with remdesivir increases the efficacy of remedesivir in NHBE cells infected with CoV2.

### Example 5: Probenecid Reduces SARS-CoV-2 B.1.1.7 Plaque Formation in vitro

### Materials and Methods

### Genomic RNA from SARS-Related Coronavirus 2, Isolate USA/CA_CDC_5574/2020 (Lineage B.1.1.7) (BEI Resources, Catalog No. NR-55244)

SARS-CoV-2, isolate USA/CA_CDC_5574/2020 was isolated from a nasopharyngeal swab on December 29, 2020 in San Diego County, California, USA (see, e.g., GISAID website). Under the nomenclature system introduced by GISAID (Global Initiative on Sharing All Influenza Data), SARS-CoV-2, isolate USA/CA_CDC_5574/2020 is assigned lineage B.1.1.7 and GISAID clade GR using Phylogenetic Assignment of Named Global Outbreak LINeages (PANGOLIN) tool (GISAID website, 3. Rambaut, et al., Nat. Microbiol. 5 (2020): 1403-1407. PubMed: 32669681; Mercatelli, et al., Front. Microbiol. (2020): doi.org/10.3389/fmicb.2020.01800. PubMed: 32793182. The complete genome of SARS-CoV-2, isolate USA/CA_CDC_5574/2020 has been sequenced (GISAID: EPI_ISL_751801).

The following mutations are present in the clinical isolate: Spike A570D, Spike D614G, Spike D1118H, Spike H69del, Spike N501Y, Spike P681H, Spike S982A, Spike T716I, Spike V70del, Spike Y145del, M (Membrane protein) V70L, N (Nucleocapsid protein) D3L, N G204R, N R203K, N S235F, NS3 T223I, NS8 (Non-structural protein 8) Q27stop, NS8 R52I, NS8 Y73C, NSP3 (Non-structural protein 3) A890D, NSP3 I1412T, NSP3 T183I, NSP6 (Non-structural protein 6) F108del, NSP6 G107del, NSP6 S106del, NSP12 (Non-structural protein 12) P323L, NSP13 (Non-structural protein 13) A454V, NSP13 K460R. One additional SNP in ORF1ab L3826F was reported in the deposited passage two virus, in comparison to the clinical specimen.

hCoV-19/Wuhan/WIV04/2019 (WIV04) is the official reference sequence employed by GISAID (EPI_ISL_402124). WIV04 was chosen because of its high-quality genome sequence and because it represented the consensus of a handful of early submissions for the betacoronavirus responsible for COVID-19. (Okada, et al., Euro Surveill., 25(8), 5 pages (2020) pii=2000097. DOI:10.2807/1560-7917.ES.2020.25.8.2000097. WIV04 is representative of and identical to the early outbreak sequences. WIV04 was isolated by the Wuhan Institute of Virology from a clinical sample of a bronchoalveolar lavage fluid (BALF) collected at the Wuhan Jinyintan Hospital in Hubei Province on 30th December 2019 from a symptomatic patient, a retailer working at the Huanan Seafood Wholesale Market.

### Time of infection assay

Vero cells were tested in 12 well format at time of infection. Probenecid was evaluated at: 100, 50, 25, 12, 6, 3, 1, 0.1, 0.01, 0.001, 0.0001, and 0.00001 µM. The assay was allowed to continue for 3 days. The cells were fixed and stained to visualize plaques. Plaques were quantified.

### Results

An *in vitro* plaque assay was conducted generally as discussed above using SARS-CoV-2, isolate USA/CA_CDC_5574/2020 as the SARS-CoV-2 test virus. This variant of the original SARS-CoV-2 isolate is classified as a variant of concern due to increased transmissibility.

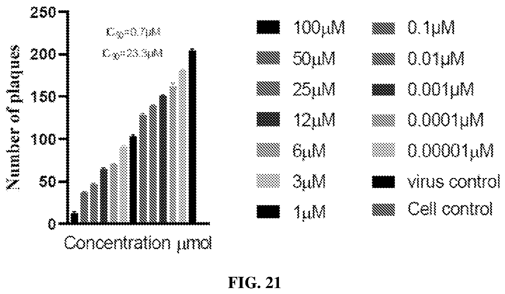

The data in Figure 21 shows that probenecid has a similar reduction of viral titer in SARS-CoV-2 isolate hu/USA/CA_CDC_5574/2020 compared to the SARS-CoV-2 strain 2019-nCoV/USA-WA1/2020 as illustrated in Example 1 and the associated Figures.

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific embodiments of the invention described herein. Such equivalents are intended to be encompassed by the following claims.

## Claims

1. A compound for use in treating a severe acute respiratory syndrome coronavirus (SARS-CoV-2) infection in a subject, wherein the compound is selected from probenecid, metabolites thereof, analogs thereof, and pharmaceutically acceptable salts of any of the foregoing, wherein the metabolite or analog thereof is
an acyl glucuronide of probenecid,
a β-ether glucuronide of probenecid,
dl-p-(N-propyl-N-2-hydroxypropylsulfamoyl)benzoic acid,
p-(N-Propyl-N-3-hydroxy¬propyl¬sulfamoyl)¬benzoic acid,
p-(N-propyl-N-3-propionitrilosulfamoyl)benzoic acid,
p-(N-propyl-N-2-carboxy¬¬ethyl¬sulfamoyl)¬benzoic acid,
p-(N-propylsulfamoyl)benzoic acid,
p-(N,N-penta-methylene-sulfamoyl)-benzoic acid (piperidyl analog),
p-(N-propyl-N-2-propenyl-sulfamoyl)-benzoic acid,
p-(N-propyl-N-2-oxopropylsulfamoyl)benzoic acid,
p-sulfamoylbenzoic acid,
p-(N-methylsulfamoyl)benzoic acid,
p-(N-ethylsulfamoyl)benzoic acid,
p-(N-hexylsulfamoyl)benzoic acid,
p-(N-cyclohexylsulfamoyl)benzoic acid,
p-(N-dimethylsulfamoyl)benzoic acid,
p-(N-methyl,N-ethylsulfamoyl)benzoic acid,
p-(N,N-diethylsulfamoyl)benzoic acid,
p-(N-ethyl,N-propylsulfamoyl)benzoic acid,
p-(N,N-diiso¬propyl¬sulfamoyl)benzoic acid,
p-(N,N-dibutylsulfamoyl)benzoic acid,
o-hydroxy-p-(N-dipropyl¬sulfamoyl)benzoic acid,
o-methoxy-p-(N-dipropylsulfamoyl)benzoic acid,
o-nitro-p-(N,N-dipropylsulfamoyl)benzoic acid,
m-nitro-p-(N,N-dipropyl-sulfamoyl)benzoic acid or
m-methyl-p-(N,N-dipropylsulfamoyl)benzoic acid.

2. The compound for use as in claim 1, wherein the subject has one or more symptoms of a disease, disorder, or illness associated with the SARS-CoV-2, optionally wherein the one or more symptoms are selected from fever, congestion in the nasal sinuses and/or lungs, runny or stuffy nose, cough, sneezing, sore throat, body aches, fatigue, shortness of breath, chest tightness, wheezing when exhaling, chills, muscle aches, headache, diarrhea, tiredness, nausea, anosmia, skin rash, and combinations thereof.

3. The compound for use as in claims 1 or 2, wherein the subject has COVID-19.

4. The compound for use as in claim 1, wherein the subject is asymptomatic.

5. A compound for use in preventing a severe acute respiratory syndrome coronavirus (SARS-CoV-2) infection comprising administering to a subject in need thereof an effective amount of a compound selected from probenecid, metabolites thereof, analogs thereof, and pharmaceutically acceptable salts of any of the foregoing, wherein the metabolites and analogs thereof is
an acyl glucuronide of probenecid,
a β-ether glucuronide of probenecid,
dl-p-(N-propyl-N-2-hydroxypropylsulfamoyl)benzoic acid,
p-(N-Propyl-N-3-hydroxy-propyl-sulfamoyl)-benzoic acid,
p-(N-propyl-N-3-propionitrilosulfamoyl)benzoic acid,
p-(N-propyl-N-2-carboxy--ethyl-sulfamoyl)-benzoic acid,
p-(N-propylsulfamoyl)benzoic acid,
p-(N,N-penta-methylene-sulfamoyl)-benzoic acid (piperidyl analog),
p-(N-propyl-N-2-propenyl-sulfamoyl)-benzoic acid,
p-(N-propyl-N-2-oxopropylsulfamoyl)benzoic acid,
p-sulfamoylbenzoic acid,
p-(N-methylsulfamoyl)benzoic acid,
p-(N-ethylsulfamoyl)benzoic acid,
p-(N-hexylsulfamoyl)benzoic acid,
p-(N-cyclohexylsulfamoyl)benzoic acid,
p-(N-dimethylsulfamoyl)benzoic acid,
p-(N-methyl,N-ethylsulfamoyl)benzoic acid,
p-(N,N-diethylsulfamoyl)benzoic acid,
p-(N-ethyl,N-propylsulfamoyl)benzoic acid,
p-(N,N-diiso-propyl-sulfamoyl)benzoic acid,
p-(N,N-dibutylsulfamoyl)benzoic acid,
o-hydroxy-p-(N-dipropyl-sulfamoyl)benzoic acid,
o-methoxy-p-(N-dipropylsulfamoyl)benzoic acid,
o-nitro-p-(N,N-dipropylsulfamoyl)benzoic acid,
m-nitro-p-(N,N-dipropyl-sulfamoyl)benzoic acid or
m-methyl-p-(N,N-dipropylsulfamoyl)benzoic acid.

6. The compound for use as in claim 5, wherein the subject has been exposed to the SARS-CoV-2.

7. The compound for use as in claims 5 or 6, wherein the subject has been in close contact with a SARS-CoV-2-infected person.

8. The compound for use as in any one of claims 1-7, wherein the SARS-CoV-2 comprises a Spike protein comprising at least 70%, 75%, 80%, 85%, 86%, 87%, 88%, 89%, 90%, 91%, 92%, 93%, 94%, 95%, 96%, 97%, 98%, 99%, or 100% sequence identity to SEQ ID NO:5, optionally, wherein the SAR-CoV-2 comprises one or more Spike protein mutations relative to SEQ ID NO:5 selected from H69, optionally deletion thereof; V70, optionally deletion thereof; G142, optionally G142D; K417, optionally K417N or K417T; E484, optionally E484K; F486, optionally F486L; and N501 mutation, optionally N501Y or N501T.

9. The compound for use as in any one of claims 1-9, wherein the SARS-CoV-2 is from the B.1.1.7, B.1.351, P.1, B.1.1.207, B.1.429, B.1.427, or B.1.525 lineage.

10. The compound for use as in any one of claims 1-9, wherein the compound is in a pharmaceutical composition further comprising a pharmaceutically acceptable carrier and/or excipient.

11. The compound for use as in any one of claims 1-10, wherein the compound is administered systemically.

12. The compound for use as in any one of claims 1-11, wherein the compound is administered, orally, parenterally, topically, or mucosally, optionally wherein the compound is administered mucosally to the lungs, nasal mucosa, or combination thereof.

13. The compound for use as in any one of claims 1-12, wherein the compound is administered in an effective amount to reduce viral replication.

14. The compound for use as in any one of claims 1-13, wherein the compound is administered at a dosage of 10 mg - 1,000 mg or 50 mg - 500 mg, optionally twice daily, optionally for 14 days.

15. The compound for use as in any one of claims 1-14, wherein the subject is a human, optionally wherein the compound is administered in a dose of 250 mg to 1,000 mg once or twice a day, .optionally wherein the compound is administered to the subject for two weeks or more.

## Patentansprüche

1. Verbindung zur Verwendung bei der Behandlung einer Infektion mit dem schweren akuten respiratorischen Syndrom-Coronavirus (SARS-CoV-2) bei einem Probanden, wobei die Verbindung aus Probenecid, dessen Metaboliten, dessen Analoga und pharmazeutisch verträglichen Salzen der vorgenannten ausgewählt ist, wobei der Metabolit oder das Analogon davon
ein Acylglucuronid von Probenecid,
ein β-Ether-Glucuronid von Probenecid,
dl-p-(N-Propyl-N-2-hydroxypropylsulfamoyl)benzoesäure,
p-(N-Propyl-N-3-hydroxypropylsulfamoyl)benzoesäure,
p-(N-Propyl-N-3-propionitrilosulfamoyl)benzoesäure,
p-(N-Propyl-N-2-carboxyethylsulfamoyl)benzoesäure,
p-(N-Propylsulfamoyl)benzoesäure,
p-(N,N-Pentamethylensulfamoyl)benzoesäure (Piperidyl-Analogon),
p-(N-Propyl-N-2-propenylsulfamoyl)benzoesäure,
p-(N-Propyl-N-2-oxopropylsulfamoyl)benzoesäure,
p-Sulfamoylbenzoesäure,
p-(N-Methylsulfamoyl)benzoesäure,
p-(N-Ethylsulfamoyl)benzoesäure,
p-(N-Hexylsulfamoyl)benzoesäure
p-(N-Cyclohexylsulfamoyl)benzoesäure,
p-(N-Dimethylsulfamoyl)benzoesäure,
p-(N-Methyl-, N-Ethylsulfamoyl)benzoesäure
p-(N,N-Diethylsulfamoyl)benzoesäure,
p-(N-Ethyl-N-propylsulfamoyl)benzoesäure,
p-(N,N-Diisopropylsulfamoyl)benzoesäure,
p-(N,N-Dibutylsulfamoyl)benzoesäure,
o-Hydroxy-p-(N-Dipropylsulfamoyl)benzoesäure,
o-Methoxy-p-(N-dipropylsulfamoyl)benzoesäure,
o-Nitro-p-(N,N-dipropylsulfamoyl)benzoesäure,
m-Nitro-p-(N,N-dipropylsulfamoyl)benzoesäure oder
m-Methyl-p-(N,N-dipropylsulfamoyl)benzoesäure ist.

2. Verbindung zur Verwendung nach Anspruch 1, wobei der Proband ein oder mehrere Symptome einer mit SARS-CoV-2 assoziierten Erkrankung, Störung oder Krankheit aufweist, wobei optional das eine oder die mehreren Symptome aus Fieber, Verstopfung der Nasennebenhöhlen und/oder Lunge, laufender oder verstopfter Nase, Husten, Niesen, Halsschmerzen, Gliederschmerzen, Müdigkeit, Atemnot, Engegefühl in der Brust, Keuchen beim Ausatmen, Schüttelfrost, Muskelschmerzen, Kopfschmerzen, Durchfall, Erschöpfung, Übelkeit, Anosmie, Hautausschlag und Kombinationen davon ausgewählt sind.

3. Verbindung zur Verwendung nach Anspruch 1 oder 2, wobei der Proband an COVID-19 erkrankt ist.

4. Verbindung zur Verwendung nach Anspruch 1, wobei der Proband asymptomatisch ist.

5. Verbindung zu Verwendung bei der Vorbeugung einer Infektion mit dem schweren akuten respiratorischen Syndrom-Coronavirus (SARS-CoV-2), umfassend die Verabreichung einer wirksamen Menge einer Verbindung, ausgewählt aus Probenecid, dessen Metaboliten, dessen Analoga und pharmazeutisch verträglichen Salzen eines der vorgenannten, an einen Probanden der diese benötigt, wobei die Metaboliten und Analoga davon:
ein Acylglucuronid von Probenecid,
ein β-Ether-Glucuronid von Probenecid,
dl-p-(N-Propyl-N-2-hydroxypropylsulfamoyl)benzoesäure,
p-(N-Propyl-N-3-hydroxypropylsulfamoyl)benzoesäure,
p-(N-Propyl-N-3-propionitrilosulfamoyl)benzoesäure,
p-(N-Propyl-N-2-carboxyethylsulfamoyl)benzoesäure,
p-(N-Propylsulfamoyl)benzoesäure,
p-(N,N-Pentamethylensulfamoyl)benzoesäure (Piperidyl-Analogon),
p-(N-Propyl-N-2-propenylsulfamoyl)benzoesäure,
p-(N-Propyl-N-2-oxopropylsulfamoyl)benzoesäure,
p-Sulfamoylbenzoesäure,
p-(N-Methylsulfamoyl)benzoesäure,
p-(N-Ethylsulfamoyl)benzoesäure,
p-(N-Hexylsulfamoyl)benzoesäure
p-(N-Cyclohexylsulfamoyl)benzoesäure,
p-(N-Dimethylsulfamoyl)benzoesäure,
p-(N-Methyl-, N-Ethylsulfamoyl)benzoesäure
p-(N,N-Diethylsulfamoyl)benzoesäure,
p-(N-Ethyl-N-propylsulfamoyl)benzoesäure,
p-(N,N-Diisopropylsulfamoyl)benzoesäure,
p-(N,N-Dibutylsulfamoyl)benzoesäure,
o-Hydroxy-p-(N-Dipropylsulfamoyl)benzoesäure,
o-Methoxy-p-(N-dipropylsulfamoyl)benzoesäure,
o-Nitro-p-(N,N-dipropylsulfamoyl)benzoesäure,
m-Nitro-p-(N,N-dipropylsulfamoyl)benzoesäure oder
m-Methyl-p-(N,N-dipropylsulfamoyl)benzoesäure sind.

6. Verbindung zur Verwendung nach Anspruch 5, wobei der Proband SARS-CoV-2 ausgesetzt wurde.

7. Verbindung zur Verwendung nach Anspruch 5 oder 6, wobei der Proband in engem Kontakt mit einer mit SARS-CoV-2 infizierten Person stand.

8. Verbindung zur Verwendung nach einem der Ansprüche 1-7, wobei das SARS-CoV-2 ein Spike-Protein umfasst, das mindestens 70 %, 75 %, 80 %, 85 %, 86 %, 87 %, 88 %, 89 %, 90 %, 91 %, 92 %, 93 %, 94 %, 95 %, 96 %, 97 %, 98 %, 99 % oder 100 % Sequenzidentität mit SEQ-ID-NR:5 aufweist, wobei das SARS-CoV-2 optional eine oder mehrere Spike-Protein-Mutationen in Bezug auf SEQ-ID-NR:5 umfasst, ausgewählt aus H69, optional dessen Deletion; V70, gegebenenfalls deren Deletion; G142, gegebenenfalls G142D; K417, gegebenenfalls K417N oder K417T; E484, gegebenenfalls E484K; F486, gegebenenfalls F486L; und N501-Mutation, gegebenenfalls N501Y oder N501T.

9. Verbindung zur Verwendung nach einem der Ansprüche 1-9, wobei das SARS-CoV-2 aus der Linie B.1.1.7, B.1.351, P.1, B.1.1.207, B.1.429, B.1.427 oder B.1.525 stammt.

10. Verbindung zur Verwendung nach einem der Ansprüche 1-9, wobei die Verbindung in einer pharmazeutischen Zusammensetzung verwendet wird, ferner umfassend einen pharmazeutisch verträglichen Träger und/oder Hilfsstoff.

11. Verbindung zur Verwendung nach einem der Ansprüche 1-10, wobei die Verbindung systemisch verabreicht wird.

12. Verbindung zur Verwendung nach einem der Ansprüche 1-11, wobei die Verbindung oral, parenteral, topisch oder mukosal verwendet wird, wobei die Verbindung optional mukosal an die Lunge, die Nasenschleimhaut oder eine Kombination davon verwendet wird.

13. Verbindung zur Verwendung nach einem der Ansprüche 1-12, wobei die Verbindung in einer wirksamen Menge verabreicht wird, um die Virusreplikation zu reduzieren.

14. Verbindung zur Verwendung nach einem der Ansprüche 1-13, wobei die Verbindung verwendet wird in einer Dosierung von 10 mg bis 1.000 mg oder 50 mg bis 500 mg, gegebenenfalls zweimal täglich, gegebenenfalls über einen Zeitraum von 14 Tagen, verabreicht wird.

15. Verbindung zur Verwendung nach einem der Ansprüche 1-14, wobei der Proband ein Mensch ist, gegebenenfalls wobei die Verbindung verwendet wird in einer Dosis von 250 mg bis 1.000 mg einmal oder zweimal täglich, gegebenenfalls wobei die Verbindung dem Probanden zwei Wochen lang oder länger verabreicht wird.

## Revendications

1. Composé destiné à être utilisé dans le traitement d'une infection par le coronavirus du syndrome respiratoire aigu sévère (SARS-CoV-2) chez un sujet, dans lequel le composé est sélectionné parmi le probénécide, les métabolites de celui-ci, les analogues de celui-ci et les sels pharmaceutiquement acceptables de l'un quelconque des éléments précédents, dans lequel le métabolite ou l'analogue de celui-ci est un acylglucuronide de probénécide,
un β-étherglucuronide de probénécide
acide dl-p-(N-propyl-N-2-hydroxypropylsulfamoyl)benzoïque,
acide p-(N-Propyl-N-3-hydroxypropylsulfamoyl)benzoïque,
acide p-(N-propyl-N-3-propionitrilosulfamoyl)benzoïque,
acide p-(N-propyl-N-2-carboxyéthylsulfamoyl)benzoïque,
acide p-(N-propylsulfamoyl)benzoïque,
acide p-(N,N-pentaméthylènesulfamoyl)benzoïque (analogue pipéridyle),
acide p-(N-propyl-N-2-propénylsulfamoyl)benzoïque,
acide p-(N-propyl-N-2-oxopropylsulfamoyl)benzoïque,
acide p-sulfamoylbenzoïque,
acide p-(N-méthylsulfamoyl)benzoïque,
acide p-(N-éthylsulfamoyl)benzoïque,
acide p-(N-hexylsulfamoyl)benzoïque,
acide p-(N-cyclohexylsulfamoyl)benzoïque,
acide p-(N-diméthylsulfamoyl)benzoïque,
acide p-(N-méthyl,N-éthylsulfamoyl)benzoïque,
acide p-(N,N-diéthylsulfamoyl)benzoïque,
acide p-(N-éthyl,N-propylsulfamoyl)benzoïque,
acide p-(N,N-diisopropylsulfamoyl)benzoïque,
acide p-(N,N-dibutylsulfamoyl)benzoïque,
acide o-hydroxy-p-(N-dipropylsulfamoyl)benzoïque,
acide o-méthoxy-p-(N-dipropylsulfamoyl)benzoïque,
acide o-nitro-p-(N,N-dipropylsulfamoyl)benzoïque,
acide m-nitro-p-(N,N-dipropylsulfamoyl)benzoïque ou
acide m-méthyl-p-(N,N-dipropylsulfamoyl)benzoïque.

2. Composé destiné à être utilisé selon la revendication 1, dans lequel le sujet présente un ou plusieurs symptômes d'une maladie, d'un trouble ou d'une affection associés au SARS-CoV-2, éventuellement dans lequel ledit ou lesdits symptômes sont sélectionnés parmi la fièvre, congestion des sinus nasaux et/ou des poumons, écoulement nasal ou congestion nasale, toux, éternuements, mal de gorge, courbatures, fatigue, essoufflement, oppression thoracique, respiration sifflante à l'expiration, frissons, douleurs musculaires, mal de tête, diarrhée, épuisement, nausée, anosmie, éruption cutanée et des combinaisons de ceux-ci.

3. Composé destiné à être utilisé selon les revendications 1 ou 2, dans lequel le sujet est atteint de la COVID-19.

4. Composé destiné à être utilisé selon la revendication 1, dans lequel le sujet est asymptomatique.

5. Composé destiné à être utilisé dans la prévention d'une infection par le coronavirus du syndrome respiratoire aigu sévère (SARS-CoV-2) comprenant l'administration à un sujet qui en a besoin d'une quantité efficace d'un composé sélectionné parmi le probénécide, les métabolites de celui-ci, les analogues de celui-ci et les sels pharmaceutiquement acceptables de l'un quelconque des éléments précédents, dans lequel les métabolites et les analogues de celui-ci sont :
un acylglucuronide de probénécide,
un β-étherglucuronide de probénécide
acide dl-p-(N-propyl-N-2-hydroxypropylsulfamoyl)benzoïque,
acide p-(N-Propyl-N-3-hydroxypropylsulfamoyl)benzoïque,
acide p-(N-propyl-N-3-propionitrilosulfamoyl)benzoïque,
acide p-(N-propyl-N-2-carboxyéthylsulfamoyl)benzoïque,
acide p-(N-propylsulfamoyl)benzoïque,
acide p-(N,N-pentaméthylènesulfamoyl)benzoïque (analogue pipéridyle),
acide p-(N-propyl-N-2-propénylsulfamoyl)benzoïque,
acide p-(N-propyl-N-2-oxopropylsulfamoyl)benzoïque,
acide p-sulfamoylbenzoïque,
acide p-(N-méthylsulfamoyl)benzoïque,
acide p-(N-éthylsulfamoyl)benzoïque,
acide p-(N-hexylsulfamoyl)benzoïque,
acide p-(N-cyclohexylsulfamoyl)benzoïque,
acide p-(N-diméthylsulfamoyl)benzoïque,
acide p-(N-méthyl,N-éthylsulfamoyl)benzoïque,
acide p-(N,N-diéthylsulfamoyl)benzoïque,
acide p-(N-éthyl,N-propylsulfamoyl)benzoïque,
acide p-(N,N-diisopropylsulfamoyl)benzoïque,
acide p-(N,N-dibutylsulfamoyl)benzoïque,
acide o-hydroxy-p-(N-dipropylsulfamoyl)benzoïque,
acide o-méthoxy-p-(N-dipropylsulfamoyl)benzoïque,
acide o-nitro-p-(N,N-dipropylsulfamoyl)benzoïque,
acide m-nitro-p-(N,N-dipropylsulfamoyl)benzoïque ou
acide m-méthyl-p-(N,N-dipropylsulfamoyl)benzoïque.

6. Composé destiné à être utilisé selon la revendication 5, dans lequel le sujet a été exposé au SARS-CoV-2.

7. Composé destiné à être utilisé selon les revendications 5 ou 6, dans lequel le sujet a été en contact étroit avec une personne infectée par le SARS-CoV-2.

8. Composé destiné à être utilisé selon l'une quelconque des revendications 1 à 7, dans lequel le SARS-CoV-2 comprend une protéine Spike comprenant au moins 70 %, 75 %, 80 %, 85 %, 86 %, 87 %, 88 %, 89 %, 90 %, 91 %, 92 %, 93 %, 94 %, 95 %, 96 %, 97 %, 98 %, 99 % ou 100 % d'identité de séquence par rapport à SEQ ID NO:5, éventuellement dans lequel le SARS-CoV-2 comprend une ou plusieurs mutations de la protéine Spike par rapport à SEQ ID NO:5 sélectionnées parmi H69, éventuellement la délétion de celle-ci ; V70, éventuellement la délétion de celle-ci ; G142, éventuellement G142D ; K417, éventuellement K417N ou K417T ; E484, éventuellement E484K ; F486, éventuellement F486L ; et la mutation N501, éventuellement N501Y ou N501T.

9. Composé destiné à être utilisé selon l'une quelconque des revendications 1 à 9, dans lequel le SARS-CoV-2 provient de la lignée B.1.1.7, B.1.351, P.1, B.1.1.207, B.1.429, B.1.427 ou B.1.525.

10. Composé destiné à être utilisé selon l'une quelconque des revendications 1 à 9, dans lequel le composé se trouve dans une composition pharmaceutique comprenant en outre un support et/ou un excipient pharmaceutiquement acceptable.

11. Composé destiné à être utilisé selon l'une quelconque des revendications 1 à 10, dans lequel le composé est administré par voie systémique.

12. Composé destiné à être utilisé selon l'une quelconque des revendications 1 à 11, dans lequel le composé est administré par voie orale, parentérale, topique ou muqueuse, éventuellement dans lequel le composé est administré par voie muqueuse aux poumons, à la muqueuse nasale ou à une combinaison de ceux-ci.

13. Composé destiné à être utilisé selon l'une quelconque des revendications 1 à 12, dans lequel le composé est administré en une quantité efficace pour réduire la réplication virale.

14. Composé destiné à être utilisé selon l'une quelconque des revendications 1 à 13, dans lequel le composé est administré à une dose de 10 mg à 1 000 mg ou de 50 mg à 500 mg, éventuellement deux fois par jour, éventuellement pendant 14 jours.

15. Composé destiné à être utilisé selon l'une quelconque des revendications 1 à 14, dans lequel le sujet est un être humain, éventuellement dans lequel le composé est administré en une dose de 250 mg à 1 000 mg une ou deux fois par jour, éventuellement dans lequel le composé est administré au sujet pendant deux semaines ou plus.
